# EUROPÄISCHE PATENTSCHRIFT

(11) **EP 2 300 417 B1**
(45) Veröffentlichungstag und Bekanntmachung des Hinweises auf die Patenterteilung: **22.07.2015**
(21) Anmeldenummer: 09738222.0
(22) Anmeldetag: 30.04.2009
(51) Int. Cl.: C07C 227/16, C07C 229/30, C07D 231/14

(54) **VERFAHREN ZUR HERSTELLUNG VON 2-(AMINOMETHYLIDEN)-4,4-DIFLUOR-3-OXOBUTTERSÄUREESTERN**
PROCESS FOR PREPARING 2-(AMINOMETHYLIDENE)-4,4-DIFLUORO-3-OXOBUTYRIC ESTERS
PROCÉDÉ DE FABRICATION D'ESTERS DE L'ACIDE 2-(AMINOMÉTHYLIDÈNE)-4,4-DIFLUORO-3-OXOBUTYRIQUE

(30) Priorität: 02.05.2008 EP 08155612
(43) Veröffentlichungstag der Anmeldung: 30.03.2011
(73) Patentinhaber: BASF SE, 67056 Ludwigshafen (DE)
(72) Erfinder: ZIERKE, Thomas, 67459 Böhl-Iggelheim (DE); MAYWALD, Volker, 67071 Ludwigshafen (DE); RACK, Michael, 69214 Eppelheim (DE); SMIDT, Sebastian Peer, 68723 Oftersheim (DE); KEIL, Michael, 67251 Freinsheim (DE); WOLF, Bernd, 67136 Fußgönheim (DE); KORADIN, Christopher, 67067 Ludwigshafen (DE)
(86) Internationale Anmeldenummer: PCT/EP2009/055283
(87) Internationale Veröffentlichungsnummer: WO 2009/133178

(56) Entgegenhaltungen:
- EP-A- 2 042 482
- EP-A- 2 072 497
- WO-A-2005/042468
- US-A- 5 498 624

## Beschreibung

Die vorliegende Erfindung betrifft ein Verfahren zur Herstellung von 2-(Aminomethyliden)-4,4-difluor-3-oxobuttersäureestern, und ihre Verwendung in dem Verfahren zur Herstellung der difluormethylsubstituierten Pyrazol-4-ylcarbonsäuren und deren Ester. Die vorliegende Erfindung betrifft auch neue 2-(Aminomethyliden)-4,4-difluor-3-oxobuttersäureester.

Die WO 92/12970 beschreibt (3-Difluormethyl-1-methylpyrazol-4-yl)carboxamide und deren Verwendung als Fungizid. Die Herstellung erfolgt ausgehend von einem 4,4-Difluor-3-oxobuttersäureester, der sukzessive mit Triethylorthoformiat und mit Methylhydrazin umgesetzt wird, wobei man einen 3-Difluormethyl-1-methylpyrazol-4-carbonsäureester erhält. Dieser wird anschließend zur entsprechenden Carbonsäure verseift. Diese wird zum entsprechenden Säurechlorid und anschließend mit einem geeigneten Amin zum entsprechenden Amid umgesetzt. Die Bereitstellung des als Ausgangsverbindung benötigten 4,4-Difluor-3-oxobuttersäureesters ist jedoch vergleichsweise kostspielig und schwierig.

Die WO 2005/044804 beschreibt Alkylester fluormethylsubstituierter heterocyclischer Carbonsäuren sowie deren Herstellung durch Halogenaustausch an entsprechenden chlormethylsubstituierten heterocyclischen Carbonsäureestern unter Verwendung von Fluorierungsmitteln. Die Verwendung von Fluorierungsmitteln ist jedoch kostspielig und stellt spezielle Anforderungen an zu treffende Sicherheitsmaßnahmen und die verwendeten Apparaturen.

Die WO 2005/042468 beschreibt die Herstellung von 2-(Dialkylaminomethyliden)-4,4-dihalo-3-oxobuttersäureester durch Umsetzung von Dialkylaminoacrylsäureestern mit Dihaloessigsäurehalogeniden in Gegenwart einer Base. Um die Bildung von Dihaloketenen zu verhindern werden hier insbesondere wässrige Lösungen von Alkali- und Erdalkalihydroxiden als Base verwendet. Bei dieser Umsetzung werden jedoch keine zufriedenstellenden Ausbeuten erhalten. Die erhaltenen 2-(Dialkylaminomethyliden)-4,4-dihalo-3-oxobuttersäureester werden mit C₁-C₄-Alkylhydrazinen zu den entsprechenden N-alkylierten dihalomethylsubstituierten Pyrazol-4-ylcarbonsäureestern umgesetzt. Bei dieser Umsetzung wird jedoch keine zufriedenstellende Selektivität der 3-Dihalomethylverbindung gegenüber der entsprechenden 5-Dihalomethylverbindung erhalten. Die anschließende Trennung der beiden entstandenen Isomere ist verhältnismäßig aufwändig.

Der vorliegenden Erfindung liegt somit die Aufgabe zugrunde, ein weiteres Verfahren zur Herstellung von (3-Difluormethylpyrazol-4-yl)carbonsäureestern und deren Derivaten bereitzustellen, deren Ausgangsverbindungen mit geringem Aufwand und mit hohen Ausbeuten bereitgestellt werden können. Aus diesen Ausgangsverbindungen sollen sich weiterhin die (3-Difluormethylpyrazol-4-yl)carbon-säureester mit hoher Ausbeute herstellen lassen, wobei eine möglichst hohe Selektivität gegenüber der üblicherweise als Nebenreaktion auftretenden Reaktion zum (5-Difluormethylpyrazol-4-yl)carbonsäureester erreicht werden soll.

Überraschenderweise wurde gefunden, dass diese Aufgabe durch ein Verfahren zur Herstellung von Verbindungen der Formel (I) gelöst wird, 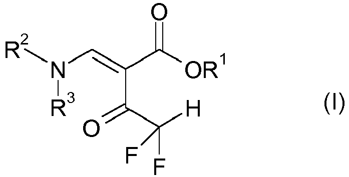 worin
- R¹: für C₁-C₆-Alkyl, C₁-C₆-Haloalkyl, C₂-C₆-Alkenyl, C₃-C₁₀-Cycloalkyl oder Benzyl steht, wobei die beiden letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen;
- R²: gemeinsam mit R³ und dem Stickstoffatom, an das die beiden Reste gebunden sind, für einen gegebenenfalls substituierten 5- bis 10-gliedrigen heterocyclischen Rest stehen, der neben dem Stickstoffatom weitere 1, 2 oder 3 Heteroatome ausgewählt unter O, N und S als Ringglieder umfassen kann;
bei dem man
eine Verbindung der Formel (II), 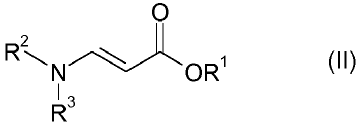 worin R¹, R² und R³ eine der zuvor gegebenen Bedeutungen aufweisen; mit Difluoracetylfluorid umsetzt.

Durch dieses erfindungsgemäße Verfahren lassen sich mit geringem Aufwand und mit hohen Ausbeuten besonders geeignete Ausgangsverbindungen für ein Verfahren zur Herstellung von (3-Difluormethylpyrazol-4-yl)carbonsäureestern bereitstellen.

Die Verbindungen der Formel (I) lassen sich mit einer guten Ausbeute bereitstellen und eignen sich in besonderem Maß zur Umsetzung mit N-substituierten Hydrazinverbindungen zu (3-Difluormethylpyrazol-4-yl)carbonsäureestern, speziell bezüglich Ausbeute und Selektivität des gewünschten Isomers gegenüber dem Nebenprodukt (5-Difluormethyl-pyrazol4-yl)carbonsäureester.

Die hier und im Folgenden in den Formeln gezeigte E-Konfiguration der C=C-Doppelbindung in den Verbindungen I und II stellt nur eine mögliche Ausgestaltung der Verbindungen I und II dar. Die Erfindung bezieht sich sowohl auf das gezeigte E-Isomer als auch das Z-Isomer und insbesondere Gemische der Isomere.

Die bei der Definition der Variablen verwendeten Begriffe für organische Gruppen sind, wie beispielsweise der Ausdruck "Halogen", Sammelbegriffe, die stellvertretend für die einzelnen Mitglieder dieser Gruppen organischer Einheiten stehen.

Das Präfix Cₓ-C_{y} bezeichnet im jeweiligen Fall die Anzahl möglicher Kohlenstoffatome.

Der Begriff "Halogen" bezeichnet jeweils Fluor, Brom, Chlor oder Iod, speziell Fluor, Chlor oder Brom.

Der Begriff "C₁-C₆-Alkyl", wie hierin und in den Alkyleinheiten von C₁-C₆-Alkoxy verwendet, bezeichnet eine gesättigte, geradkettige oder verzweigte Kohlenwasserstoffgruppe, umfassend 1 bis 6 Kohlenstoffatome, speziell 1 bis 4 Kohlenstoffatome, beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, 1,1-Dimethylpropyl, 1,2-Dimethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl und deren Isomere. C₁-C₄-Alkyl umfasst beispielsweise Methyl, Ethyl, Propyl, 1-Methylethyl, Butyl, 1-Methylpropyl, 2-Methylpropyl oder 1,1-Dimethylethyl.

Der Begriff "C₁-C₆-Alkoxy" beschreibt geradkettige oder verzweigte gesättigte Alkylgruppen, umfassend 1 bis 6 Kohlenstoffatome, die über ein Sauerstoffatom gebunden sind. Beispiele für C₁-C₆-Alkoxy umfassen, wie Methoxy, Ethoxy, n-Propoxy, 1-Methylethoxy, n-Butoxy, 1-Methylpropoxy, 2-Methylpropoxy, 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy. Beispiele für C₁-C₄-Alkoxy umfassen Methoxy, Ethoxy, n-Propoxy, 1-Methylethoxy, n-Butoxy, 1-Methylpropoxy, 2-Methylpropoxy und 1,1-Dimethylethoxy.

Der Begriff "C₁-C₆-Haloalkyl", wie hierin und in den Haloalkyleinheiten von C₁-C₆-Haloalkoxy verwendet, beschreibt geradkettige oder verzweigte Alkylgruppen mit 1 bis 6 Kohlenstoffatomen, wobei die Wasserstoffatome dieser Gruppen teilweise oder vollständig durch Halogenatome ersetzt sind. Beispiele hierfür sind C₁-C₄-Haloalkyl, wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl.

Der Begriff "C₁-C₆-Haloalkoxy" beschreibt geradkettige oder verzweigte gesättigte Haloalkylgruppen, umfassend 1 bis 6 Kohlenstoffatome, die über ein Sauerstoffatom gebunden sind. Beispiele hierfür sind C₁-C₄-Haloalkoxy, wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy und Pentafluorethoxy.

Der Begriff "C₂-C₆-Alkenyl" beschreibt geradkettige und verzweigte ungesättigte Kohlenwasserstoffgruppen, umfassend 2 bis 6 Kohlenstoff Atome und wenigstens eine Kohlenstoff-Kohlenstoff-Doppelbindung, wie beispielsweise Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl 1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Der Begriff "C₃-C₁₀-Cycloalkyl" wie hierin verwendet, beschreibt mono-, bi- oder tricyclische Kohlenwasserstoffgruppen, umfassend 3 bis 10 Kohlenstoffatome, speziell 3 bis 6 Kohlenstoffatome. Beispiele monocyclischer Gruppen umfassen Cyclopropyl, Cyclobutyl, Cyclopentyl, Cyclohexyl, Cycloheptyl oder Cyclooctyl. Beispiele bicyclischer Gruppen umfassen Bicyclo[2.2.1]heptyl, Bicyclo[3.1.1]heptyl, Bicyclo[2.2.2]octyl und Bicyclo[3.2.1]octyl. Beispiele tricyclischer Gruppen sind Adamantyl und Homoadamantyl.

Der Begriff "5- bis 10-gliedrigen heterocyclischen Rest" steht im Zusammenhang mit der Definition der Gruppe -NR²R³ für eine stickstoffhaltige mono- oder bicyclische Gruppe mit 5, 6, 7, 8, 9 oder 10 Ringgliedern, die über das N-Atom an den übrigen Teil der Verbindung der Formel (I) bzw. (II) gebunden ist, neben dem Stickstoffatom weitere 1, 2 oder 3 Heteroatome ausgewählt unter O, N und S als Ringglieder aufweisen kann und unsubstituiert ist oder 1, 2 oder 3 Substituenten aufweisen kann. Die Substituenten sind, wenn diese an ein Kohlenstoffatom des heterocyclischen Restes gebunden sind, bevorzugt ausgewählt unter Halogen, CN, C₁-C₄-Alkyl, C₁-C₄-Haloalkyl, C₁-C₄-Alkoxy und C₁-C₄-Haloalkoxy, und, wenn diese an ein weiteres Stickstoffatom des heterocyclischen Restes gebunden sind, bevorzugt ausgewählt unter C₁-C₄-Alkyl und C₁-C₄-Haloalkyl. Beispiele 5- bis 10-gliedriger heterocyclischer Reste sind Pyrrol-1-yl, Pyrrolidin-1-yl, Oxazolidin-3-yl, Thiazolidin-3-yl, Imidazol-1-yl, Imidazolin-1-yl, 3-Methylimidazolin-1-yl, 3-Ethylimidazolin-1-yl, 3-Propylimidazolin-1-yl, 3-(1-Methylethyl)imidazolin-1-yl, 3-Butylimidazolin-1-yl, 3-(1,1-Dimethylethyl)imidazolin-1-yl, Pyrazol-1-yl, Pyrazolidin-1-yl, 2-Methylpyrazolidin-1-yl, 2-Ethylpyrazolidin-1-yl, 2-Propylpyrazolidin-1-yl, 2-(1-Methylethyl)pyrazolidin-1-yl, 2-Butylpyrazolidin-1-yl, 2-(1,1-Dimethylethyl)pyrazolidin-1-yl, Piperidin-1-yl, Morpholin-4-yl, Thiamorpholin-4-yl, Piperazin-1-yl, 4-Methylpiperazin-1-yl, 4-Ethylpiperazin-1-yl, 4-Propylpiperazin-1-yl, 4-(1-Methylethyl)piperazin-1-yl, 4-Butylpiperazin-1-yl, 4-(1,1-Dimethylethyl)piperazin-1-yl, Indol-1-yl, Indolin-1-yl, Isoindol-1-yl, Isoindolin-1-yl, Indazol-1-yl, Indazolin-1-yl, 2-Methylindazolin-1-yl, Indazolin-2-yl und 1-Methylindazolin-1-yl, wobei die zuvor genannten heterocyclischen Gruppen unsubstituiert sind oder 1, 2 oder 3 der Ringkohlenstoffatome einen Substituenten ausgewählt unter Halogen, CN, Nitro, C₁-C₄-Alkyl, C₁-C₄-Haloalkyl, C₁-C₄-Alkoxy und C₁-C₄-Haloalkoxy tragen.

Die Kohlenstoff-Kohlenstoff-Doppelbindung in den Verbindungen der Formeln (I) und (II) kann E- oder Z-Konfiguration aufweisen (bzw. cis- oder trans-Konfiguration, bezogen auf die relative Anordnung der Gruppe NR²R³ und der Gruppe -C(O)OR¹).

Die hierin beschriebenen Umsetzungen werden in für derartige Reaktionen üblichen Reaktionsgefäßen durchgeführt, wobei die Reaktionsführung sowohl kontinuierlich als auch diskontinuierlich ausgestaltet werden kann. In der Regel wird man die jeweiligen Reaktionen drucklos durchführen. Die Reaktionen können jedoch auch unter Druck durchgeführt werden.

In den Verbindungen der Formeln (I) und (II) steht R¹ bevorzugt für C₁-C₆-Alkyl, C₃-C₁₀-Cycloalkyl oder Benzyl, wobei die beiden letztgenannten Reste unsubstituiert sind oder 1, 2 oder 3 Substituenten aufweisen. Bevorzugt steht R¹ in den Verbindungen der Formel (I) und (II) für C₁-C₄-Alkyl, C₃-C₆-Cycloalkyl oder Benzyl. Ganz besonders bevorzugt steht R¹ in den Verbindungen der Formeln (I) und (II) für C₁-C₄-Alkyl.

In den Verbindungen der Formeln (I) und (II) steht R² gemeinsam mit R³ und dem Stickstoffatom, an das die beiden Reste gebunden sind, für einen gegebenenfalls substituierten 5- bis 10-gliedrigen heterocyclischen Rest stehen, der neben dem Stickstoffatom weitere 1, 2 oder 3 Heteroatome ausgewählt unter O, N und S als Ringglieder umfassen kann, d. h. die Gruppe NR²R³ steht für ein N-gebundenen 5- bis 10-gliedrigen heterocyclischen Rest. Dies bezieht sich sowohl auf die Verbindungen der Formeln (I) und (II) an sich, als auch auf deren Verwendung in den erfindungsgemäßen Verfahren zur Herstellung von (3-Difluormethylpyrazol-4-yl)carbonsäureestern.

In den Verbindungen der Formeln (I) und (II) steht die Gruppe NR²R³ bevorzugt für einen gesättigten, gegebenenfalls substituierten, 5- oder 6-gliedrigen heterocyclischen Rest, der neben dem Stickstoffatom ein weiteres Heteroatom ausgewählt unter O, N und S als Ringglieder aufweisen kann. Insbesondere steht die Gruppe NR²R³ für Pyrrolidin-1-yl, Oxazolidin-3-yl, 3-Methylimidazolin-1-yl, Piperidin-1-yl, Morpholin-4-yl oder 4-Methylpiperazin-1-yl. Speziell steht die Gruppe NR²R³ in den Verbindungen der Formeln (I) und (II) für Piperidin-1-yl, Morpholin-4-yl oder 4-Methylpiperazin-1-yl.

Üblicherweise setzt man in dem erfindungsgemäßen Verfahren zur Herstellung von Verbindungen der Formel (I) die Verbindung der Formel (II) in einer Menge von 0,2 bis 3 Mol, bevorzugt von 0,3 bis 1,5 Mol, insbesondere von 0,5 bis 1,0 Mol und besonders bevorzugt von 0,9 bis 1,0 Mol, jeweils bezogen auf 1 Mol Difluoracetylfluorid, ein.

Die Umsetzung erfolgt, indem man die Ausgangsverbindungen, d. h. die Verbindung der Formel (II) und das Difluoracetylfluorid, vorzugsweise in einem geeigneten Lösungsmittel in einem Reaktionsgefäß miteinander in Kontakt bringt, wobei man in der Regel die Verbindung der Formel (II) und gegebenenfalls das Lösungsmittel im Reaktionsgefäß vorlegt.

Üblicherweise führt man die Umsetzung der Verbindung der Formel (II) mit Difluoracetylfluorid bei einer Temperatur im Bereich von -70 bis +50 °C, bevorzugt von -30 bis +20 °C und besonders bevorzugt von -10 bis 0 °C, durch. In einer speziellen Ausführungsform wird zu Beginn eine Temperatur von -50 bis -10 °C eingestellt und im Verlauf der Reaktion auf 10 bis 40 °C, insbesondere Raumtemperatur, erhöht.

Üblicherweise führt man die Umsetzung der Verbindung der Formel (II) mit Difluoracetylfluorid bei Atmosphärendruck durch. Aufgrund des niedrigen Siedepunkts von Difluoracetylfluorid kann es jedoch in Abhängigkeit der gewählten Reaktionstemperatur von Vorteil sein die Umsetzung unter höherem Druck durchzuführen. Geeignete Reaktionsdrücke liegen beispielsweise in einem Bereich von 0,5 bis 10 bar. Geeignete druckfeste Reaktoren sind dem Fachmann ebenfalls bekannt und werden z. B. in Ullmanns Enzyklopädie der technischen Chemie, Bd. 1, 3. Auflage, 1951, S. 769 ff. beschrieben.

Vorzugsweise erfolgt in dem erfindungsgemäßen Verfahren zur Herstellung von Verbindungen der Formel (I) die Umsetzung der Verbindung der Formel (II) mit Difluoracetylfluorid im Wesentlichen wasserfrei, d. h. in einem trockenen organischen Lösungsmittel.

Hier und im Folgenden bedeutet trockenes Lösungsmittel, dass das Lösungsmittel einen Wassergehalt von weniger als 500 ppm und insbesondere von weniger als 100 ppm aufweist.

Beispiele geeigneter organischer Lösungsmittel sind unpolare, aprotische Lösungsmitteln, z.B. aromatische Kohlenwasserstoffe, wie Benzol, Toluol, Xylole, oder (cyclo)aliphatische Kohlenwasserstoffe, wie Hexan, Cyclohexan und dergleichen, sowie Mischungen der zuvor genannten Lösungsmittel.

Beispiele geeigneter organischer Lösungsmittel sind ebenso aprotische polare Lösungsmittel, z. B. cyclische oder acyclische Ether, wie Diethylether, tert.-Butylmethylether (MTBE), Diisopropylether, Cyclopentylmethylether, Tetrahydrofuran (THF) oder Dioxan, cyclische oder acyclische Amide, wie Dimethylformamid, Dimethylacetamid, N-Methylpyrrolidon, Harnstoffe, wie N,N'-Dimethyl-N,N'-ethylenharnstoff (DMEU), N,N'-Dimethyl-N,N'-propylenharnstoff (DMPU) oder Tetramethylharnstoff, oder aliphatische Nitrile, wie Acetonitril oder Propionitril, sowie Mischungen der zuvor genannten Lösungsmittel.

Ebenso geeignet sind Mischungen der vorgenannten unpolaren, aprotischen organischen Lösungsmittel mit polaren, aprotischen Lösungsmitteln.

In einer speziellen Ausführungsform des erfindungsgemäßen Verfahrens wird die Verbindung der Formel (II) mit Difluoracetylfluorid ohne Zusatz einer von der Verbindung der Formel (II) verschiedenen Base umgesetzt.

In einer weiteren speziellen Ausführungsform des erfindungsgemäßen Verfahrens wird die Verbindung der Formel (II) mit Difluoracetylfluorid in Gegenwart einer von der Verbindung der Formel (II) verschiedenen Base umsetzt.

Als von der Verbindung der Formel (II) verschiedene Basen sind organische Basen geeignet, z. B. acyclische tertiäre Amine, z.B. Tri-C₁-C₆-alkylamine wie Trimethylamin, Triethylamin, Tributylamin, Diisopropylethylamin, tert.-Butyldimethylamin, N-C₃-C₆-cycloalkyl-N,N-di-C₁-C₆-alkylamine oder N,N-bis-C₃-C₆-cycloalkyl-N-C₁-C₆-alkylamine wie Ethyldicyclohexylamin, cyclische tertiäre Amine, z.B. N-C₁-C₆-Alkyl-Stickstoffheterocyclen wie N-Methylpyrrolidin, N-Methylpiperidin, N-Methylmorpholin, N,N'-Dimethylpiperazin, Pyridinverbindungen wie Pyridin, Collidin, Lutidin oder 4-Dimethylaminopyridin, sowie bicyclische Amine, wie Diazabicycloundecen (DBU) oder Diazabicyclononen (DBN).

Ebenso sind als von der Verbindung der Formel (II) verschiedene Basen anorganische Verbindungen geeignet, z.B. Alkalimetall- und Erdalkalimetallcarbonate, wie Lithiumcarbonat oder Calciumcarbonat, Alkalimetallhydrogencarbonate, wie Natriumhydrogencarbonat, Alkalimetall- und Erdalkalimetalloxide, wie Lithiumoxid, Natriumoxid, Calciumoxid oder Magnesiumoxid, Alkalimetall- und Erdalkalimetallhydride, wie Lithiumhydrid, Natriumhydrid, Kaliumhydrid oder Calciumhydrid, oder Alkalimetallamide, wie Lithiumamid, Natriumamid oder Kaliumamid.

Bevorzugt ist die von der Verbindung der Formel (II) verschiedene Base in dem erfindungsgemäßen Verfahren zur Herstellung von Verbindungen der Formel (I) ausgewählt unter organischen Basen. Besonders bevorzugt ist die Base ausgewählt unter acyclischen tertiären Aminen, speziell Triethylamin.

Die von der Verbindung der Formel (II) verschiedene Base kann sowohl in etwa äquimolaren Mengen, bezogen auf die Verbindung (II), z.B. in einer Menge von etwa 0,8 bis 1,2 Mol, bezogen auf ein Mol der Verbindung (II), als auch in katalytischen Mengen, bezogen auf die Verbindung (II), z.B. in einer Menge von etwa 0,001 bis 0,2 Mol, bezogen auf ein Mol der Verbindung (II), eingesetzt werden. Die Base kann aber auch in großem Überschuss bezogen auf die Verbindung der Formel (II), z.B. als Lösungsmittel eingesetzt werden.

Üblicherweise wird man die Verbindung der Formel (I) unter etwa pH-neutralen Bedingungen, d.h. bei einem pH-Wert im Bereich von 4 bis 10, oder unter nicht wässrigen Bedingungen isolieren, um eine übermäßige Hydrolyse der Gruppe -C(O)OR¹ zu verhindern.

Eine Isolierung der Verbindungen der Formel (I) ist jedoch für die im Folgenden beschriebene Umsetzung zu dem entsprechenden Pyrazol-4-ylcarbonsäureester nicht erforderlich. Vielmehr hat es sich als vorteilhaft erwiesen, auf eine Isolierung der Verbindung (I) zu verzichten und diese als Rohprodukt oder in Form des in dem erfindungsgemäßen Verfahren erhaltenen Reaktionsgemisches zu den entsprechenden Pyrazol-4-ylcarbonsäureestern umzusetzen.

Durch das erfindungsgemäßen Verfahren werden die Verbindungen der Formel (I) ausgehend von den Verbindungen der Formel (II) in guten bis sehr guten Ausbeuten erhalten, d.h. in der Regel in Ausbeuten von wenigstens 70 % und häufig von wenigstens 80 %.

Die Verbindungen der allgemeinen Formel (II) sind kommerziell erhältlich oder können analog zu bekannten Verbindungen, wie beispielsweise in Schema 1 oder 2 veranschaulicht, hergestellt werden.

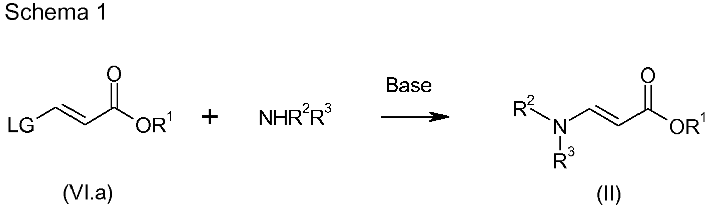

Schema 1 zeigt die Herstellung von Verbindungen der Formel (II) durch Reaktion eines α,β-ungesättigten Esters der Formel (VI.a), worin R¹ eine der zuvor gegebenen Bedeutungen aufweist und LG für eine Abgangsgruppe, wie beispielsweise eine Alkoxygruppe, beispielsweise C₁-C₆-Alkoxy, mit einem Amin der Formel NHR²R³, worin R² und R³ eine der zuvor gegebenen Bedeutungen aufweisen, in Gegenwart einer Base, wie beispielsweise K₂CO₃. Geeignete Methoden zur Durchführung dieser Reaktion sind dem Fachmann bekannt.

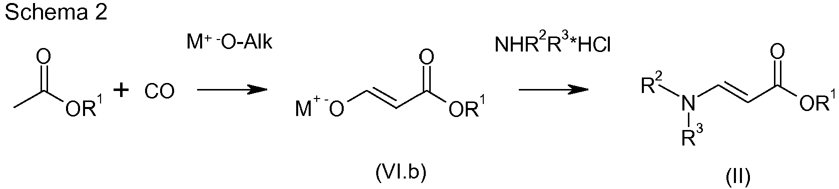

Alternativ lassen sich Verbindungen der Formel (II), analog der EP 0 388 744, durch Umsetzung eines β-Hydroxyacrylsäureestersalzes der Formel (VI.b), worin R¹ eine der zuvor genannten Bedeutungen aufweist und M⁺ beispielsweise für ein Alkalimetallkation, wie Na⁺ oder K⁺, steht, mit einem Ammoniumchlorid der Formel NHR²R³*HCl bereitstellen.

Verbindungen der Formel (VI.b) können beispielsweise durch Umsetzung des entsprechenden Essigsäureesters mit CO und einem Alkoholat der Formel M⁺⁻O-Alk, worin Alk beispielsweise für C₁-C₄-Alkyl steht, bereitgestellt werden.

Die Amine der Formel NHR²R³ fallen bei der Umsetzung der Verbindungen der Formel (I) zu Difluormethylpyrazolylcarbonsäureestern als Nebenprodukt an und können nach erfolgter Umsetzung vorteilhafterweise zurückgewonnen und gegebenenfalls in deren Hydrochloride NHR²R³*HCl überführt werden, um erneut zur Bereitstellung von Verbindungen der Formel (II) gemäß Schema 1 oder 2 verwendet zu werden.

Die durch das erfindungsgemäße Verfahren hergestellten Verbindungen der Formel (I), worin R² gemeinsam mit R³ und dem Stickstoffatom, an das die beiden Reste gebunden sind, für einen heterocyclischen Rest stehen, sind ebenfalls neu. Demzufolge betrifft ein weiterer Gegenstand der Erfindung die Verbindungen der Formel (I), 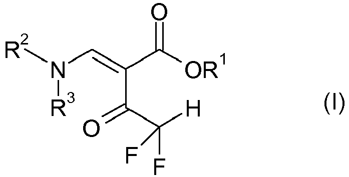 worin R¹ eine der zuvor gegebenen Bedeutungen aufweist und R² gemeinsam mit R³ und dem Stickstoffatom, an das die beiden Reste gebunden sind, für einen gegebenenfalls substituierten 5- bis 10-gliedrigen heterocyclischen Rest stehen, der neben dem Stickstoffatom weitere 1, 2 oder 3 Heteroatome ausgewählt unter O, N und S als Ringglieder umfassen kann.

Die Verbindungen der Formel (I), worin R² gemeinsam mit R³ und dem Stickstoffatom, an das die beiden Reste gebunden sind, für einen heterocyclischen Rest stehen, eignen sich in besonders vorteilhafter Weise zur Herstellung difluormethylsubstituierter Pyrazol-4-ylcarbonsäureester. Demzufolge betrifft ein weiterer Gegenstand der Erfindung ein Verfahren zur Herstellung difluormethylsubstituierter Pyrazol-4-ylcarbonsäureester der Formel (III), 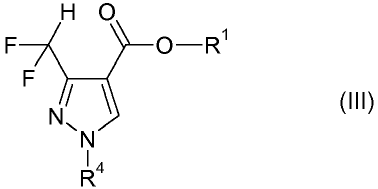 worin
- R¹: für C₁-C₆-Alkyl, C₁-C₆-Haloalkyl, C₂-C₆-Alkenyl, C₃-C₁₀-Cycloalkyl oder Benzyl steht, wobei die beiden letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen; und
- R⁴: für Wasserstoff, C₁-C₆-Alkyl, C₁-C₆-Haloalkyl, C₃-C₁₀-Cycloalkyl, Phenyl oder Benzyl steht, wobei die drei letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen;
bei dem man
a) eine Verbindung der Formel (I) bereitstellt, 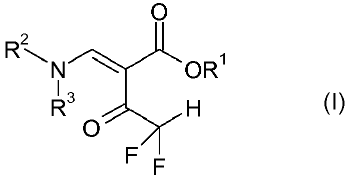 worin R¹ eine der zuvor gegebenen Bedeutungen aufweist und R² gemeinsam mit R³ und dem Stickstoffatom, an das die beiden Reste gebunden sind, für einen gegebenenfalls substituierten 5- bis 10-gliedrigen heterocyclischen Rest stehen, der neben dem Stickstoffatom weitere 1, 2 oder 3 Heteroatome ausgewählt unter O, N und S als Ringglieder umfassen kann; und
b) die in Schritt a) bereitgestellte Verbindung der Formel (I) mit einer Hydrazin-Verbindung der Formel (IV) umsetzt,

   R⁴HN-NH₂ (IV)

   worin R⁴ eine der zuvor gegebenen Bedeutungen besitzt.

Das erfindungsgemäße Verfahren zur Herstellung von Verbindungen der Formel (III) ist mit einer Reihe von Vorteilen verbunden. Das erfindungsgemäße Verfahren liefert die Verbindungen der Formel (III) mit hoher Ausbeute. Zudem wird, wenn R⁴ eine von H verschiedene Bedeutung aufweist, die Verbindung der Formel (III) mit hoher Selektivität gegenüber dem als Nebenprodukt gebildeten (5-Difluormethylpyrazol-4-yl)carbonsäureester hergestellt. Somit kann auf eine aufwändige Trennung der Isomerengemische verzichtet oder diese zumindest begrenzt werden. Außerdem lässt sich das erfindungsgemäße Verfahren sowohl wasserfrei als auch in Gegenwart von Wasser unter gleichzeitigem Erhalt zufriedenstellender Ausbeuten und Überschüsse der Verbindung der Formel (III) durchführen.

Bevorzugte weist die Gruppe R¹ in den Verbindungen der Formel (III) eine der Bedeutungen auf, die zuvor als bevorzugte Bedeutungen der Gruppen R¹ in den Verbindungen der Formeln (I) und (II) genannt worden sind.

In einer speziellen Ausführungsform des erfindungsgemäßen Verfahrens, weist die Gruppe R⁴ in den Verbindungen der Formeln (III) und (IV) eine von Wasserstoff verschiedene Bedeutung auf. Die Verbindungen der Formel (III), worin R⁴ eine von Wasserstoff verschiedene Bedeutung aufweist, lassen sich durch das erfindungsgemäße Verfahren mit besonders hoher Selektivität gegenüber den entsprechenden 5-Difluormethylpyrazol-4-ylcarbonsäureestern herstellen.

Bevorzugt steht die Gruppe R⁴ in den Verbindungen der Formeln (III) und (IV) für C₁-C₄-Alkyl, C₃-C₆-Cycloalkyl, Phenyl oder Benzyl, wobei die drei letztgenannten Reste unsubstituiert sind oder 1, 2 oder 3 Substituenten aufweisen, die unabhängig voneinander unter Halogen, C₁-C₄-Alkyl und C₁-C₄-Alkoxy ausgewählt sind. Besonders Bevorzugt steht R⁴ in den Verbindungen der Formeln (III) und (IV) für C₁-C₆-Alkyl, insbesondere für C₁-C₄-Alkyl und speziell für Methyl.

Bevorzugt erfolgt die Bereitstellung einer Verbindung der Formel (I) (Schritt a) in dem erfindungsgemäßen Verfahren zur Herstellung von Verbindungen der Formel (III) durch das zuvor beschriebene Verfahren zur Herstellung von Verbindungen der Formel (I).

In einer speziellen Ausführungsform des erfindungsgemäßen Verfahrens zur Herstellung von Verbindungen der Formel (III) setzt man ein Reaktionsgemisch mit der Hydrazinverbindung der Formel (IV) um, wobei das Reaktionsgemisch eine Verbindung der Formel (I) enthält, das durch das zuvor beschriebene Verfahren bereitgestellt wurde, ohne dabei die Verbindung der Formel (I) zuvor zu isolieren.

### Schritt b)

Vorzugsweise setzt man die Hydrazinverbindung der Formel (IV), in äquimolaren Mengen oder im Überschuss bezogen auf die Verbindung der Formel (I) ein, wobei ein größerer Überschuss an Verbindung (IV), z. B. mehr als 20 Mol-%, in der Regel nicht erforderlich ist. Vorzugsweise setzt man 1,0 bis 1,2 Mol, insbesondere etwa 1,01 bis 1,15 Mol der Hydrazinverbindung (IV) je Mol der Verbindung (I) ein.

Bevorzugt handelt es sich bei der Hydrazinverbindung der Formel (IV) um ein C₁-C₆-Alkylhydrazin und insbesondere um ein C₁-C₄-Alkylhydrazin; speziell handelt es sich bei der Verbindung der allgemeinen Formel (IV) um Methylhydrazin.

Steht R⁴ in den Verbindungen der Formel (III) für Wasserstoff, verwendet man bevorzugt Hydrazin-Hydrat als Verbindung der Formel (IV).

Bei der Umsetzung der Verbindung der Formel (I) mit der Hydrazinverbindung (IV) wird man üblicherweise so vorgehen, dass man die Hydrazinverbindung der Formel (IV) in einem geeigneten Lösungsmittel vorlegt, die gewünschte Reaktionstemperatur einstellt und anschließend die Verbindung der Formel (I), gegebenenfalls in Form einer Lösung und/oder eines bei der Bereitstellung erhaltenen Reaktionsgemisches, zugibt.

Vorzugsweise legt man die Hydrazinverbindung der Formel (IV) als Lösung in einem organischen Lösungsmittel oder in einem Lösungsmittel/Wasser-Gemisch vor. Alternativ kann man auch so vorgehen, dass man die Hydrazinverbindung der Formel (IV), vorzugsweise als Lösung in einem organischen Lösungsmittel oder in einem Lösungsmittel/Wasser-Gemisch, zu der Verbindung der Formel (I), gegebenenfalls in Form einer Lösung in einem organischen Lösungsmittel oder in einem Lösungsmittel/WasserGemisch, gibt.

Für die Umsetzung der Verbindung der Formel (I) mit der Hydrazinverbindung (IV) geeignete organische Lösungsmittel sind beispielsweise protisch polare Lösungsmittel, wie aliphatische Alkohole mit vorzugsweise 1 bis 4 Kohlenstoffatomen, speziell Methanol, Ethanol, n-Propanol, Isopropanol, n-Butanol, Isobutanol oder tert.-Butanol, unpolare, aprotische Lösungsmittel, z.B. aromatische Kohlenwasserstoffe, wie Benzol, Toluol, Xylole, Mesitylen, Cumol, Chlorbenzol, Nitrobenzol oder tert.-Butylbenzol, aprotische polare Lösungsmittel, wie cyclische oder acyclische Ether, speziell Diethylether, tert.-Butylmethylether (MTBE), Cyclopentylmethylether, Tetrahydrofuran (THF) oder Dioxan, cyclische oder acyclische Amide, speziell Dimethylformamid, Dimethylacetamid, N-Methylpyrrolidon, Harnstoffe, wie N,N'-Dimethyl-N,N'-ethylenharnstoff (DMEU), N,N'-Dimethyl-N,N'-propylenharnstoff (DMPU) oder Tetramethylharnstoff, oder aliphatische Nitrile, speziell Acetonitril oder Propionitril, oder Mischungen der zuvor genannten Lösungsmittel.

Die Umsetzung der Verbindung der Formel (I) mit der Hydrazinverbindung (IV) kann gegebenenfalls in Gegenwart einer Base durchgeführt werden.

Geeignete Basen hierfür sind organische Basen , z. B. die vorgenannten acyclischen tertiären Amine, wie Trimethylamin, Triethylamin, Diisopropylethylamin, tert.-Butyldimethylamin oder Ethyldicyclohexylamin, die vorgenannten cyclischen tertiären Amine, wie N-Methylpyrrolidin, N-Methylpiperidin, N-Methylmorpholin, N,N'-Dimethylpiperazin, Pyridin, Collidin, Lutidin oder 4-Dimethylaminopyridin, oder bicyclische Amine, wie Diazabicycloundecen (DBU) oder Diazabicyclononen (DBN).

Ebenso als Basen geeignet sind anorganische Verbindungen, z.B. Alkalimetall- und Erdalkalimetallhydroxide, wie Natriumhydroxid, Kaliumhydroxid oder Calciumhydroxid, Alkalimetall- und Erdalkalimetalloxide, wie Lithiumoxid, Natriumoxid, Calciumoxid oder Magnesiumoxid, Alkalimetall- und Erdalkalimetallcarbonate, wie Lithiumcarbonat oder Calciumcarbonat, Alkalimetallhydrogencarbonate, wie Natriumhydrogencarbonat, Alkalimetall- und Erdalkalimetallhydride, wie Lithiumhydrid, Natriumhydrid, Kaliumhydrid oder Calciumhydrid, oder Alkalimetallamide, wie Lithiumamid, Natriumamid oder Kaliumamid.

Die Base kann sowohl in etwa äquimolaren Mengen, bezogen auf die Verbindung (I), z.B. in einer Menge von etwa 0,8 bis 1,2 Mol, bezogen auf 1 Mol der Verbindung (I), als auch in katalytischen Mengen, bezogen auf die Verbindung (I), z.B. in einer Menge von etwa 0,001 bis 0,2 Mol, bezogen auf 1 Mol der Verbindung (I), eingesetzt werden. Die Base kann aber auch in großem Überschuss bezogen auf die Verbindung der Formel (II), z.B. als Lösungsmittel eingesetzt werden.

Durch die Zugabe einer Base können gegebenenfalls größere Überschüsse der 3-Difluormethylpyrazol-4-ylcarbonsäureester der Formel (III) bezogen auf die als Nebenprodukt gebildeten 5-Difluormethylpyrazol-4-ylcarbonsäureester erzielt werden.

In einer speziellen Ausführungsform des erfindungsgemäßen Verfahrens zur Herstellung von Verbindungen der Formel (III), erfolgt die Umsetzung der Verbindung der Formel (I) mit der Hydrazinverbindung der Formel (IV) in Gegenwart von Wasser. Hierbei ist bereits ein geringer Wassergehalt des Reaktionsgemisches von 1000 ppm ausreichend. Das bei der Umsetzung freigesetzte Wasser ist bei der Angabe des Wassergehalts nicht berücksichtigt.

In der Regel wird der Wassergehalt des Reaktionsgemisches 50 Vol.-%, häufig 30 Vol.-%, insbesondere 15 Vol.-% nicht überschreiten und liegt häufig im Bereich von 0,1 bis 50 Vol.-%, vorzugsweise im Bereich von 0,5 bis 30 Vol.-%, insbesondere im Bereich von 1 bis 15 Vol.-%.

Üblicherweise führt man die Umsetzung der Verbindung der Formel (I) in Gegenwart von Wasser bei Temperaturen von -80 bis +100 °C durch. In einer speziellen Ausführungsform wird zu Beginn der Umsetzung eine Temperatur von -50 bis +20 °C, insbesondere von -15 bis +10 °C, eingestellt und im Verlauf der Reaktion auf eine Temperatur von +10 bis +40 °C, insbesondere auf Raumtemperatur, erhöht.

Führt man die Umsetzung der Verbindung der Formel (I) in Gegenwart von Wasser und einer Base durch, ist die Base vorzugsweise unter den zuvor genannten anorganischen Verbindungen, speziell unter den zuvor genannten Alkalimetall- oder Erdalkalimetallbasen und insbesondere unter Alkalimetallhydroxiden oder Erdalkalimetallhydroxiden, wie NaOH oder KOH, ausgewählt. Bezüglich der eingesetzten Mengen gilt das zuvor Gesagte.

Durch das erfindungsgemäße Verfahren werden die Verbindungen der Formel (III), bei Umsetzung einer Verbindung der Formel (I) in Gegenwart von Wasser, in guten Ausbeuten erhalten, d.h. in der Regel in Ausbeuten von wenigstens 60 % und häufig von wenigstens 70 %. Zudem werden, wenn R⁴ eine von H verschiedene Bedeutung aufweist, die Verbindungen der Formel (III) in dieser Ausführungsform des Verfahrens mit hoher Selektivität gegenüber den entsprechenden 5-Difluormethylpyrazol-4-ylcarbonsäureestern erhalten, d.h. in der Regel in einem Verhältnis von 3-Difluormethylpyrazol-4-ylcarbonsäureester zu 5-Difluormethylpyrazol-4-ylcarbonsäureester von wenigstens 2,5 : 1 und häufig von wenigstens 5 : 1. Durch die Gegenwart einer geeigneten Base werden häufig Verhältnisse von wenigstens 10 : 1 oder sogar 20 :1 erzielt.

In einer weiteren speziellen Ausführungsform des erfindungsgemäßen Verfahrens zur Herstellung von Verbindungen der Formel (III), erfolgt die Umsetzung der Verbindung der Formel (I) mit der Hydrazinverbindung der Formel (IV) im Wesentlichen wasserfrei, d. h. das Reaktionsgemisch weist einen Wassergehalt von weniger als 500 ppm und insbesondere von weniger als 100 ppm auf. Das bei der Umsetzung freigesetzte Wasser ist bei der Angabe des Wassergehalts nicht berücksichtigt.

Üblicherweise führt man das erfindungsgemäße Verfahren, worin die Umsetzung einer Verbindung der Formel (I) im Wesentlichen wasserfrei erfolgt, bei Temperaturen von -80 bis +100 °C durch. In einer speziellen Ausführungsform wird zu Beginn der Umsetzung eine Temperatur von -80 bis -10 °C, insbesondere von -60 bis -30 °C, eingestellt und im Verlauf der Reaktion auf eine Temperatur von +10 bis +40 °C, insbesondere auf Raumtemperatur, erhöht.

Führt man das erfindungsgemäße Verfahren, worin die Umsetzung einer Verbindung der Formel (I) im Wesentlichen wasserfrei erfolgt, in Gegenwart einer Base durch, ist diese vorzugsweise unter Erdalkalimetall- und Alkalimetallcarbonaten und den zuvor genannten organischen Basen, insbesondere unter den organischen Basen und speziell unter den zuvor genannten Pyridinen und acyclische tertiären Amine, wie Pyridin oder Triethylamin, ausgewählt. Bezüglich der verwendeten Menge gilt das zuvor Gesagte.

Durch das erfindungsgemäßen Verfahren, worin die Umsetzung einer Verbindung der Formel (I) im Wesentlichen wasserfrei erfolgt, werden die Verbindungen der Formel (III) in guten bis sehr guten Ausbeuten erhalten, d.h. in der Regel in Ausbeuten von wenigstens 80 % und häufig von wenigstens 90 %. Zudem werden, wenn R⁴ eine von H verschiedene Bedeutung aufweist, die Verbindungen der Formel (III) in dieser Ausführungsform des Verfahrens mit sehr großer Selektivität gegenüber den entsprechenden 5-Difluormethylpyrazol-4-ylcarbonsäureestern erhalten, d.h. in der Regel in einem Verhältnis von 3-Difluormethylpyrazol-4-ylcarbonsäureester zu 5-Difluormethylpyrazol-4-ylcarbonsäureester von wenigstens 10 : 1 und häufig von wenigstens 20 : 1.

Die Aufarbeitung der erhaltenen Reaktionsgemische und die Isolierung der Verbindung der Formel (III) erfolgt in üblicher Weise, beispielsweise durch Entfernen des Lösungsmittels, z. B. unter vermindertem Druck, durch eine wässrige, extraktive Aufarbeitung oder durch eine Kombination dieser Maßnahmen. Eine weitere Reinigung kann beispielsweise durch Kristallisation oder durch Chromatographie erfolgen. Häufig fällt das Produkt bereits in einer Reinheit an, die weitere Reinigungsverfahren überflüssig macht.

Bei der Umsetzung der Verbindungen der Formel (I) zu Difluormethylpyrazolylcarbonsäureestern fallen die Amine der Formel NHR²R³ als Nebenprodukt an. Diese können durch geeignete dem Fachmann bekannte Maßnahmen isoliert und zur Bereitstellung von Verbindungen der Formel (II) verwendet zu werden. Die Isolierung der Amine der Formel NHR²R³ kann beispielsweise durch übliche Trennverfahren, wie Fällung durch Einstellen des pH-Werts oder durch Extraktion, erfolgen.

Die Verbindungen der Formel (III) lassen sich durch Hydrolyse in die entsprechenden difluormethylsubstituierten Pyrazol-4-ylcarbonsäuren überführen.

Demzufolge betrifft ein weiterer Gegenstand der Erfindung ein Verfahren zur Herstellung einer Pyrazol-4-carbonsäure der Formel (V), 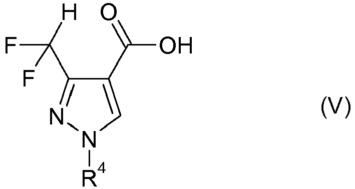 worin R⁴ eine der zuvor gegebenen Bedeutungen besitzt, umfassend die Bereitstellung einer Verbindung der Formel (III), wie zuvor definiert, durch ein erfindungsgemäßes Verfahren und die Hydrolyse der Esterfunktion in der Verbindung der Formel (III), wodurch man die Pyrazolcarbonsäure der Formel (V) erhält.

In einer speziellen Ausführungsform des erfindungsgemäßes Verfahren zur Herstellung von Verbindungen der Formel (V), wird ein durch ein erfindungsgemäßes Verfahren zur Herstellung von Verbindungen der Formel (III) bereitgestelltes Reaktionsgemisch, enthaltend eine Verbindung der Formel (III), ohne vorherige Isolierung der Verbindung der Formel (III) hydrolysiert.

Die Hydrolyse der Esterfunktion in der Verbindung (III) kann unter Säurekatalyse oder basisch oder in sonstiger Weise erfolgen. Die Verbindung (III) kann als solche, d. h. nach Isolierung eingesetzt werden. Es ist jedoch auch möglich das in Schritt b) erhaltene Reaktionsgemisch, gegebenenfalls nach Abtrennung flüchtiger Bestandteile, wie Lösungsmittel, ohne Isolierung der Verbindung der Formel (III) umzusetzen.

Bei der basischen Hydrolyse der Verbindung (III) wird man üblicherweise die Verbindung der Formel (III) mit einem Alkalimetallhydroxid oder Erdalkalimetallhydroxid, wie Natriumhydroxid, Kaliumhydroxid oder Lithiumhydroxid, bevorzugt mit einer wässrigen Alkalimetallhydroxid-Lösung oder Erdalkalimetallhydroxid-Lösung, speziell einer wässrigen NaOH-Lösung oder einer wässrigen KOH-Lösung, bis zur vollständigen Hydrolyse des Esters behandeln, vorzugsweise unter Erwärmen.

Bei der basischen Hydrolyse liegt das molare Verhältnis von Verbindung der Formel (III) zu Base üblicherweise im Bereich von 0,8 : 1 bis 1 : 10 und ist insbesondere etwa äquimolar, z.B. im Bereich von 0,8 :1 bis 1,2 : 1, jedoch kann auch ein größerer Überschuss an Base, z. B. bis zu 5 Mol je Mol Verbindung (III), von Vorteil sein.

Üblicherweise erfolgt die basische Hydrolyse in einem Verdünnungs- bzw. Lösungsmittel. Geeignete Verdünnungs- bzw. Lösungsmittel sind neben Wasser auch Mischungen organischer Lösungsmittel, die gegenüber Alkali stabil sind, mit Wasser. Beispiele für alkalistabile organische Lösungsmittel sind insbesondere die zuvor genannten C₁-C₄-Alkohole sowie die zuvor genannten acyclischen und cyclischen Ether.

Ebenso geeignet sind Gemische unpolarer Lösungsmittel, z. B. aromatischer Kohlenwasserstoffe, wie Benzol, Toluol, Xylole, oder (cyclo)aliphatischer Kohlenwasserstoffe, wie Hexan, Cyclohexan und dergleichen, mit Wasser.

Vorzugsweise führt man die basische Hydrolyse bei Temperaturen von 20 bis 100 °C durch. In der Regel ist die obere Temperaturgrenze der Siedepunkt des verwendeten Lösungsmittels bei druckloser Reaktionsführung. Vorzugsweise wird man eine Reaktionstemperatur von 100 °C und insbesondere von 90 °C nicht überschreiten. Die Reaktionsdauer ist hierbei von der Reaktionstemperatur, der Konzentration und der Stabilität der jeweiligen Esterverbindung abhängig. Im Allgemeinen werden die Reaktionsbedingungen so gewählt, dass die Reaktionszeit im Bereich von 0,5 bis 12 h und insbesondere im Bereich von 1 bis 6 h liegt.

Die saure Hydrolyse der Estergruppe der Verbindung (III) kann in Analogie zu bekannten sauren Esterhydrolysen durchgeführt werden, d. h. in Gegenwart katalytischer oder stöchiometrischer Mengen einer Säure und Wasser (siehe z. B. J. March, Advanced Organic Chemistry, 2nd Ed., 334-338, McGraw-Hill, 1977 und dort zitierte Literatur). Häufig wird man die Umsetzung in einer Mischung aus Wasser und einem aprotischen organischen Lösungsmittel, beispielsweise einem Ether, wie zuvor genannt, durchführen. Beispiele für geeignete Säuren sind Halogenwasserstoffsäuren, Schwefelsäure, organische Sulfonsäuren, wie p-Toluolsulfonsäure oder Methansulfonsäure, Phosphorsäure sowie saure lonentauscherharze und dergleichen.

Geeignete Hydrolysekatalysatoren sind außerdem Alkalimetalliodide, Lithiumiodid, Trimethyliodsilan oder Mischungen von Trimethylchlorsilan mit Alkalimetalliodiden wie Lithium-, Natrium- oder Kaliumiodid.

Die Isolierung der Säure (V) erfolgt durch übliche Trennverfahren, wie beispielsweise durch Fällung durch Einstellen des pH-Werts oder durch Extraktion.

Bei der Verwendung von Gemischen unpolarer Lösungsmittel mit Wasser als Reaktionsmedium, hat es sich als besonders vorteilhaft erwiesen, die Säure der Formel (V) unter basischen pH-Bedingungen als Lösung in der wässrigen Phase abzutrennen und anschließend durch Einstellen eines sauren pH-Werts aus der wässrigen Lösung als Feststoff auszufällen. Sofern die Verbindungen der Formel (III) in Form eines durch ein erfindungsgemäßes Verfahren bereitgestelltes Reaktionsgemisch ohne vorherige Isolierung der Verbindung der Formel (III) durch Hydrolyse umgesetzt wird, wird bei dieser vorgehensweise das als Nebenprodukt der Vorstufe angefallene Amin der Formel NHR²R³ als Lösung in der abgetrennten organischen Phase erhalten und kann zur Bereitstellung von Verbindungen der Formel (II) verwendet zu werden.

Die Verbindungen der allgemeinen Formel (III) und (V) eignen sich für die Synthese einer Vielzahl als Wirkstoff interessanter Verbindungen, wie beispielsweise zur Herstellung von 3-Difluormethylpyrazol-4-carboxamiden, insbesondere von 3-Difluormethylpyrazol-4-carboxaniliden.

Geeignete Methoden zur Herstellung von Aniliden durch Umsetzung von Carbonsäuren oder Carbonsäurehalogeniden mit aromatischen Aminen sind dem Fachmann bekannt, z.B. aus dem eingangs zitierten Stand der Technik sowie aus J. March, Advanced Organic Chemistry, 2nd Ed., 382 f, McGraw-Hill, 1977 und Organikum, 21. Auflage Wiley-VCH, Weinheim 2001, S. 481-484 und dort zitierte Literatur.

Beispiele für 3-Difluormethylpyrazol-4-carboxamide, die sich auf diesem Wege herstellen lassen, sind:
N-(2-Bicyclopropyl-2-yl-phenyl)-3-difluormethyl-1-methylpyrazol-4-yl-carboxamid,
N-(3',4',5'-Trifluorbiphenyl-2-yl)-3-difluormethyl-1-methyl-pyrazol-4-yl-carboxamid,
N-(2',4',5'-Trifluorbiphenyl-2-yl)-3-difluormethyl-1-methyl-pyrazol-4-yl-carboxamid,
N-(3',4'-Dichlor-3-fluorbiphenyl-2-yl)-1-methyl-3-difluormethyl-1H-pyrazol-4-yl-carboxamid,
N-(3',4'-Difluor-3-fluorbiphenyl-2-yl)-1-methyl-3-difluormethyl-1H-pyrazol-4-yl-carboxamid,
N-(3'-Chlor-4'-fluor-3-fluorbiphenyl-2-yl)-1-methyl-3-difluormethyl-1H-pyrazol-4-yl-carboxamid,
N-(3',4'-Dichlor-4-fluorbiphenyl-2-yl)-1-methyl-3-difluormethyl-1H-pyrazol-4-yl-carboxamid,
N-(3',4'-Difluor-4-fluorbiphenyl-2-yl)-1-methyl-3-difluormethyl-1H-pyrazol-4-yl-carboxamid,
N-(3'-Chlor-4'-fluor-4-fluorbiphenyl-2-yl)-1-methyl-3-difluormethyl-1H-pyrazol-4-yl-carboxamid,
N-(3',4'-Dichlor-5-fluorbiphenyl-2-yl)-1-methyl-3-difluormethyl-1H-pyrazol-4-yl-carboxamid,
N-(3',4'-Difluor-5-fluorbiphenyl-2-yl)-1-methyl-3-difluormethyl-1H-pyrazol-4-yl-carboxamid,
N-(3'-Chlor-4'-fluor-5-fluorbiphenyl-2-yl)-1-methyl-3-difluormethyl-1H-pyrazol-4-yl-carboxamid,
N-[2-(1,1,2,3,3,3-Hexafluorpropoxy)-phenyl]-3-difluormethyl-1-methyl-1 H-pyrazol-4-yl-carboxamid,
N-[4'-(Trifluormethylthio)-biphenyl-2-yl]-3-difluormethyl-1-methyl-1H-pyrazol-4-yl-carboxamid,
3-(Difluormethyl)-1-methyl-N-[1,2,3,4-tetrahydro-9-(1-methylethyl)-1,4-methanonaphthalin-5-yl]-1H-pyrazol-4-yl-carboxamid,
N-(3'-Chlor-5-fluorbiphenyl-2-yl)- 3-(difluormethyl)-1-methylpyrazol-4-yl-carboxamid,
N-(4'-Chlor-5-fluorbiphenyl-2-yl)- 3-(difluormethyl)-1-methylpyrazol-4-yl-carboxamid,
N-(4'-Chlorbiphenyl-2-yl)-3-(difluormethyl)-1-methylpyrazol-4-yl-carboxamid,
N-(4'-Brombiphenyl-2-yl)-3-(difluormethyl)-1-methylpyrazol-4-yl-carboxamid,
N-(4'-Jodbiphenyl-2-yl)-3-(difluormethyl)-1-methylpyrazol-4-yl-carboxamid,
N-(3',5'-difluorbiphenyl-2-yl)-3-(difluormethyl)-1-methylpyrazol-4-yl-carboxamid,
N-(2-Chlor-4-fluor-phenyl)- 3-(difluormethyl)-1-methylpyrazol-4-yl-carboxamid,
N-(2-Brom-4-fluor-phenyl)- 3-(difluormethyl)-1-methylpyrazol-4-yl-carboxamid und
N-(2-Jod-4-fluor-phenyl)- 3-(difluormethyl)-1-methylpyrazol-4-yl-carboxamid.

Im Folgenden wird die vorliegende Erfindung anhand nicht einschränkender Beispiele näher erläutert.

### Beispiele

### 1. Herstellung von Verbindungen der Formel (I)

### Herstellungsbeispiel 1.1: 4,4-Difluor-3-oxo-2-(piperidin-1-ylmethyliden)buttersäure-ethylester (Alternative 1)

3-Piperidin-1-ylacrylsäureethylester (94%ig, 9,4 g, 48 mmol) und Triethylamin (5,2 g, 51 mmol) wurden in 100 ml Toluol vorgelegt und auf -30°C gekühlt. Anschließend wurde bei dieser Temperatur Difluoracetylfluorid (5 g, 51 mmol) eingeleitet. Das Reaktionsgemisch wurde 3h bei -30°C gerührt und anschließend innerhalb einer Stunde auf Raumtemperatur erwärmt. Das Reaktionsgemisch wurde mit vollentsalztem Wasser (50 ml) gewaschen. Nach Trennung der Phasen wurde die wässrige Phase einmal mit Toluol (100 ml) extrahiert. Die Toluolphasen wurden vereinigt, mit einer wässrigen ges. NaCl-Lösung (100 ml) gewaschen und anschließend am Vakuum vom Lösungsmittel befreit. 4,4-Difluor-3-oxo-2-(1-piperidin-1-ylmethyliden)buttersäureethylester wurde als orangefarbenes Öl erhalten (Menge: 13,0 g; Reinheit It. GC: 91,2 %; Ausbeute: 94 %). ¹H-NMR (CDCl₃): δ = 1,3 (t, 3H), 1,75 (m, 6H), 3,3 (m, 2H), 3,6 (m, 2H), 4,25 (q, 2H), 6,6 (t, 1 H), 7,85 ppm (s, 1 H).

### Herstellungsbeispiel I.2: 4,4-Difluor-3-oxo-2-(piperidin-1-ylmethyliden)buttersäure-ethylester (Alternative 2)

3-Piperidin-1-ylacrylsäureethylester (94%ig, 9,4 g, 48 mmol) wurde in Toluol (100 ml) vorgelegt und auf -30°C gekühlt. Bei dieser Temperatur wurde Difluoracetylfluorid (5,0 g, 51 mmol) in das Reaktionsgemisch eingeleitet. Anschließend wurde das Reaktionsgemisch mit Triethylamin (5,2 g, 51 mmol) versetzt. Das Reaktionsgemisch wurde 3h bei -30°C gerührt und anschließend innerhalb einer Stunde auf Raumtemperatur erwärmt. Das Reaktionsgemisch wurde mit vollentsalztem Wasser (50 ml) gewaschen. Nach Trennung der Phasen wurde die wässrige Phase einmal mit Toluol (100 ml) extrahiert. Die Toluolphasen wurden vereinigt, mit einer wässrigen ges. NaCl-Lösung (100 ml) gewaschen und anschließend am Vakuum vom Lösungsmittel befreit. 4,4-Difluor-3-oxo-2-(1-piperidin-1-ylmethyliden)buttersäureethylester wurde als orangefarbenes Öl erhalten (Menge: 12,6 g; Reinheit It. GC: 85,5%; Ausbeute: 85,5%).

### Herstellungsbeispiel I.3: 4,4-Difluor-3-oxo-2-(piperidin-1-ylmethyliden)buttersäure-ethylester (Alternative 3)

3-Piperidin-1-ylacrylsäureethylester (94%ig, 9,4 g, 48 mmol) wurde in Toluol (100 ml) vorgelegt und auf -30°C abgekühlt. Bei dieser Temperatur wurde Difluoracetylfluorid (5,0 g, 51 mmol) eingeleitet. Anschließend wurde das Reaktionsgemisch mit Triethylamin (7,7 g, 77 mmol) versetzt. Das Reaktionsgemisch wurde 3h bei -30°C gerührt und anschließend innerhalb einer Stunde auf Raumtemperatur erwärmt. Das Reaktionsgemisch wurde mit vollentsalztem Wasser (50 ml) gewaschen. Nach Trennung der Phasen wurde die wässrige Phase einmal mit Toluol (100 ml) extrahiert. Die Toluolphasen wurden vereinigt, mit einer wässrigen ges. NaCl-Lösung (100 ml) gewaschen und anschließend am Vakuum vom Lösungsmittel befreit. 4,4-Difluor-3-oxo-2-(1-piperidin-1-ylmethyliden)buttersäureethylester wurde als orangefarbenes Öl erhalten (Menge: 12,3 g; Reinheit It. GC: 85,4%; Ausbeute: 83,4%).

### Herstellungsbeispiel I.4: 4,4-Difluor-3-oxo-2-(piperidin-1-ylmethyliden)buttersäure-ethylester (Alternative 4)

3-Piperidin-1-ylacrylsäureethylester (94%ig, 9,4 g, 48 mmol) wurde in Toluol (100 ml) vorgelegt und auf -30°C abgekühlt. Bei dieser Temperatur wurde Difluoracetylfluorid (5,0 g, 51 mmol) eingeleitet. Anschließend wurde das Reaktionsgemisch mit Tributylamin (9,5 g, 51 mmol) versetzt. Das Reaktionsgemisch wurde 3h bei -30°C gerührt und anschließend innerhalb einer Stunde auf Raumtemperatur erwärmt. Das Reaktionsgemisch wurde mit vollentsalztem Wasser (50 ml) gewaschen. Nach Trennung der Phasen wurde die wässrige Phase einmal mit Toluol (100 ml) extrahiert. Die Toluolphasen wurden vereinigt, mit einer wässrigen ges. NaCl-Lösung (100 ml) gewaschen und anschließend am Vakuum vom Lösungsmittel befreit. 4,4-Difluor-3-oxo-2-(1-piperidin-1-ylmethyliden)buttersäureethylester wurde als orangefarbenes Öl erhalten (Menge: 12,7g; Reinheit It. GC: 82,15%; Ausbeute: 82,8%).

### Herstellungsbeispiel I.5: 4,4-Difluor-3-oxo-2-(piperidin-1-ylmethyliden)buttersäuremethylester

3-Piperidin-1-yl-acrylsäuremethylester (96,7%ig, 50,7 g, 289 mmol) und Triethylamin (31,07 g, 307 mol) wurden in Toluol (300 ml) vorgelegt und auf -30°C abgekühlt. Bei dieser Temperatur wurde Difluoracetylfluorid eingeleitet (30,1 g 307 mmol). Das Reaktionsgemisch wurde 3h bei -30°C gerührt und anschließend innerhalb einer Stunde auf Raumtemperatur erwärmt. Das Reaktionsgemisch wurde mit vollentsalztem Wasser (150 ml) gewaschen. Nach Trennung der Phasen wurde die wässrige Phase einmal mit Toluol (100 ml) extrahiert. Die Toluolphasen wurden vereinigt, mit einer wässrigen ges. NaCl-Lösung (100 ml) gewaschen und anschließend am Vakuum vom Lösungsmittel befreit. 4,4-Difluor-3-oxo-2-(1-piperidin-1-ylmethyliden)buttersäuremethylester wurde als orangefarbenes Öl erhalten (Menge: 69,7 g; Reinheit It. GC: 92,3%; Ausbeute: 90%). ¹H-NMR (CDCl₃): δ = 1,75 (m, 6H), 3,3 (m, 2H), 3,62 (m, 2H), 3,75 (s, 3H), 6,62 (t, 1 H), 7,89 ppm (s, 1 H).

### Herstellungsbeispiel I.6: 4,4-Difluor-3-oxo-2-(piperidin-1-ylmethyliden)buttersäuremethylester (Vergleichsbeispiel zu I.5, nicht erfindungsgemäß)

3-Piperidin-1-yl-acrylsäuremethylester (99%ig, 26,6 g, 155 mmol) und Triethylamin (17,3 g, 171 mmol) wurden in Toluol (300 ml) vorgelegt und auf -30°C abgekühlt. Bei dieser Temperatur wurde Difluoracetylchlorid eingeleitet (20 g, 171 mmol). Das Reaktionsgemisch wurde 3h bei -30°C gerührt, nochmals 200 ml Toluol zugegeben um die Suspension rührbar zu halten und anschließend innerhalb einer Stunde auf Raumtemperatur erwärmt. Das Reaktionsgemisch wurde mit vollentsalztem Wasser (200 ml) gewaschen. Nach Trennung der Phasen wurde die wässrige Phase einmal mit Toluol (100 ml) extrahiert. Die Toluolphasen wurden vereinigt, mit einer wässrigen ges. NaCl-Lösung (100 ml) gewaschen und anschließend am Vakuum vom Lösungsmittel befreit. 4,4-Difluor-3-oxo-2-(1-piperidin-1-ylmethyliden)buttersäuremethylester wurde als orangefarbenes Öl erhalten (Menge: 37,6 g; Reinheit It. quant. NMR: 82,2%; Ausbeute: 80,3%).

### Herstellungsbeispiel I.7: 4,4-Difluor-3-oxo-2-(piperidin-1-ylmethyliden)buttersäuremethylester

3-Piperidin-1-yl-acrylsäuremethylester (99%ig, 8,7 g, 51 mmol) und Pyridin (4,26 g, 54mmol) wurden in Toluol (150 ml) vorgelegt und auf -30°C abgekühlt. Bei dieser Temperatur wurde Difluoracetylfluorid eingeleitet (10 g, 61 mmol). Das Reaktionsgemisch wurde 3h bei -30°C gerührt und anschließend innerhalb einer Stunde auf Raumtemperatur erwärmt. Das Reaktionsgemisch wurde mit vollentsalztem Wasser (200 ml) gewaschen. Nach Trennung der Phasen wurde die wässrige Phase einmal mit Toluol (100 ml) extrahiert. Die Toluolphasen wurden vereinigt, mit einer wässrigen ges. NaCl-Lösung (100 ml) gewaschen und anschließend am Vakuum vom Lösungsmittel befreit. 4,4-Difluor-3-oxo-2-(1-piperidin-1-ylmethyliden)buttersäuremethylester wurde als orangefarbenes Öl erhalten (Menge: 12,0 g; Reinheit It. GC: 97,7%; Ausbeute: 93,2%).

### Herstellungsbeispiel I.8: 4,4-Difluor-3-oxo-2-(morpholin-4-ylmethyliden)buttersäure-methylester

3-Morpholin-4-yl-acrylsäuremethylester (96,3%ig, 8,5 g, 50 mmol) und Triethylamin (5,2 g, 50mmol) wurden in Toluol (250 ml) vorgelegt und auf -30°C abgekühlt. Bei dieser Temperatur wurde Difluoracetylfluorid eingeleitet (5,0 g, 50 mmol). Das Reaktionsgemisch wurde 3h bei -30°C gerührt und anschließend innerhalb einer Stunde auf Raumtemperatur erwärmt. Das Reaktionsgemisch wurde am Vakuum vom Lösungsmittel befreit. Als Rückstand wurden 15,5 g eines orangefarbenen Feststoffs erhalten, der laut quant. ¹H-NMR zu 66,4% aus 4,4-Difluor-3-oxo-2-(morpholin-4-ylmethyliden)-buttersäuremethylester bestand (Ausbeute: 82%). ¹H-NMR (CDCl₃): δ = 3,4 (m, 2H), 3,7 (m, 2H), 3,75 (s, 3H), 3,85 (m, 2H), 6,6 (t, 1 H), 7,85 ppm (s, 1 H).

### Herstellungsbeispiel I.9: 4,4-Difluor-3-oxo-2-(2,6-dimethylmorpholin-4-ylmethyliden)-buttersäuremethylester

3-(2,6-Dimethylmorpholin-4-yl)acrylsäuremethylester (93,5%ig, 17,5 g, 82 mmol) und Triethylamin (8,8 g, 87mmol) wurden in Toluol (150 ml) vorgelegt und auf -30°C abgekühlt. Bei dieser Temperatur wurde Difluoracetylfluorid eingeleitet (10,0 g 100 mmol). Das Reaktionsgemisch wurde 3h bei -30°C gerührt und anschließend innerhalb einer Stunde auf Raumtemperatur erwärmt. Das Reaktionsgemisch wurde mit vollentsalztem Wasser (100 ml) gewaschen. Nach Trennung der Phasen wurde die wässrige Phase einmal mit Toluol (100 ml) extrahiert. Die Toluolphasen wurden vereinigt, mit einer wässrigen ges. NaCl-Lösung (100 ml) gewaschen und anschließend am Vakuum vom Lösungsmittel befreit. Als Rückstand wurden 21,4 g eines orangefarbener Feststoff erhalten, der laut GC-Analytik zu 90,8% aus 4,4-Difluor-3-oxo-2-(2,6-dimethyl-morpholin-4-ylmethyliden)buttersäuremethylester bestand (Ausbeute: 85,3%). ¹H-NMR (CDCl₃): δ = 1,2 (s, 3H), 1,25 (s, 3H), 2,95 (m, 1 H), 3,2 5 (m, 1 H), 3,5 (m, 1 H), 3,75 (m, 2H), 3,8 (s, 3H), 6,6 (t, 1 H), 7,85 ppm (s, 1 H).

### 2. Herstellung von Verbindungen der Formel (III)

### Herstellungsbeispiel III.1: 3-Difluormethyl-1-methyl-1 H-pyrazol-4-ylcarbonsäureethylester (Alternative 1)

Methylhydrazin (0,33 g, 7 mmol) wurde in Toluol (50 ml) gelöst und auf -50°C abgekühlt. Bei dieser Temperatur wurde zu dem Reaktionsgemisch eine Lösung von 4,4-Difluor-3-oxo-2-(1-piperidin-1-ylmethyliden)buttersäureethylester (85,5%ig, 2,0 g, 6,5 mmol) in Toluol (50 ml) zugetropft. Das Reaktionsgemisch wurde 2 h bei -50°C gerührt und anschließend innerhalb einer Stunde auf Raumtemperatur erwärmt. Danach wurde das Reaktionsgemisch unter vermindertem Druck vom Lösungsmittel befreit. Der Rückstand wurde in Essigsäureethylester (50 ml) gelöst und mit vollentsalztem (50 ml) Wasser gewaschen. Nach Trocknen der organischen Phase über MgSO₄ wurde das Lösungsmittel unter vermindertem Druck entfernt. 3-Difluormethyl-1-methyl-1 H-pyrazol-4-ylcarbonsäureethylester wurde als Rückstand erhalten (Menge: 1,7 g; Reinheit It. GC: 76,3 % (bezogen auf 3-Isomer); Ausbeute: 97 %). Das Isomerenverhältnis von 3-Isomer zu 5-Isomer im Rückstand betrug 97 : 3. ¹H-NMR (CDCl₃): δ = 1,35 (t, 3H), 3,95 (s, 2H), 4,3 (q, 2H), 7,12 (t, 1 H), 7,9 ppm (s, 1 H).

### Herstellungsbeispiel III.2: 3-Difluormethyl-1-methyl-1 H-pyrazol-4-ylcarbonsäureethylester (Alternative 2)

Eine wässrige Lösung von Methylhydrazin (35%ig, 0,95 g, 7 mmol) wurde mit Toluol (50 ml) vermischt und auf -50°C abgekühlt. Bei dieser Temperatur wurde zu dem Reaktionsgemisch eine Lösung von 4,4-Difluor-3-oxo-2-(1-piperidin-1-ylmethyliden)-buttersäureethylester (85,5%ig, 2,0 g ,6,5 mmol) in Toluol (50 ml) zugetropft. Das Reaktionsgemisch wurde 2 h bei -50°C gerührt und anschließend innerhalb einer Stunde auf Raumtemperatur erwärmt. Danach wurde das Reaktionsgemisch unter vermindertem Druck vom Lösungsmittel befreit. Der Rückstand wurde in Essigsäureethylester (50 ml) gelöst und mit vollentsalztem Wasser (50 ml) gewaschen. Nach Trocknen der organischen Phase über MgSO₄ wurde das Lösungsmittel unter vermindertem Druck entfernt. 3-Difluormethyl-1-methyl-1 H-pyrazol-4-ylcarbonsäureethylester wurde als Rückstand erhalten (Menge: 1,7 g; Reinheit It. GC: 59,3 % (bezogen auf 3-Isomer); Ausbeute: 75,4 %). Das Isomerenverhältnis von 3-Isomer zu 5-Isomer im Rückstand betrug 76 : 24.

### Herstellungsbeispiel III.3: 3-Difluormethyl-1-methyl-1 H-pyrazol-4-ylcarbonsäureethylester (Alternative 3)

Eine wässrige Lösung von Methylhydrazin (35%ig, 0,95 g, 7 mmol) wurde mit Triethylamin (0,66 g, 6,5mol) und Toluol (50 ml) vermischt und auf -50°C abgekühlt. Bei dieser Temperatur wurde eine Lösung von 4,4-Difluor-3-oxo-2-(1-piperidin-1-ylmethyliden)-buttersäureethylester (85,5%ig, 2,0 g, 6,5 mmol) in Toluol (50 ml) zugetropft. Das Reaktionsgemisch wurde 2 h bei -50°C gerührt und anschließend innerhalb einer Stunde auf Raumtemperatur erwärmt. Danach wurde das Reaktionsgemisch unter vermindertem Druck vom Lösungsmittel befreit. Der Rückstand wurde in Essigsäureethylester (50 ml) gelöst und mit vollentsalztem Wasser (50 ml) gewaschen. Nach Trocknen der organischen Phase über MgSO₄ wurde das Lösungsmittel unter vermindertem Druck entfernt. 3-Difluormethyl-1-methyl-1 H-pyrazol-4-ylcarbonsäureethylester wurde als Rückstand erhalten (Menge: 1,8 g; Reinheit It. GC: 64,5 % (bezogen auf 3-Isomer); Ausbeute: 73,4 %). Das Isomerenverhältnis von 3-Isomer zu 5-Isomer im Rückstand betrug 84 : 16.

### Herstellungsbeispiel III.4: 3-Difluormethyl-1-methyl-1H-pyrazol-4-ylcarbonsäure-ethylester (Alternative 4)

Eine wässrige Lösung von Methylhydrazin (35%ig, 0,95 g, 7 mmol) wurde mit Molekularsieb (5,0 g) und Toluol (50 ml) vermischt und auf -50°C abgekühlt. Bei dieser Temperatur wurde eine Lösung von 4,4-Difluor-3-oxo-2-(1-piperidin-1-ylmethyliden)-buttersäureethylester (85,5%ig, 2,0 g, 6,5 mmol) in Toluol (50 ml) zugetropft. Das Reaktionsgemisch wurde 2 h bei -50°C gerührt und anschließend innerhalb einer Stunde auf Raumtemperatur erwärmt. Danach wurde das Reaktionsgemisch filtriert und unter vermindertem Druck vom Lösungsmittel befreit. Der Rückstand wurde in Essigsäureethylester (50 ml) gelöst und mit vollentsalztem Wasser (50 ml) gewaschen. Nach Trocknen der organischen Phase über MgSO₄ wurde das Lösungsmittel unter vermindertem Druck entfernt. 3-Difluormethyl-1-methyl-1H-pyrazol-4-ylcarbonsäureethylester wurde als Rückstand erhalten (Menge: 1,3 g; Reinheit It. GC: 72,9 % (bezogen auf 3-Isomer); Ausbeute: 71,0 %). Das Isomerenverhältnis von 3-Isomer zu 5-Isomer im Rückstand betrug 88 : 12.

### Herstellungsbeispiel III.5: 3-Difluormethyl-1-methyl-1 H-pyrazol-4-ylcarbonsäure-ethylester (Alternative 5)

Eine wässrige Lösung von Methylhydrazin (35%ig, 1,03 g, 8 mmol) wurde mit Natronlauge (10%ig, 2,6 g, 6,5 mmol) und Ethanol (50 ml) vermischt und auf -3 °C abgekühlt. Bei dieser Temperatur wurde eine Lösung von 4,4-Difluor-3-oxo-2-(1-piperidin-1-ylmethyliden)buttersäureethylester (85,5%ig, 2,0 g, 6,5 mmol) in Toluol (50 ml) zugetropft. Das Reaktionsgemisch wurde 2 h bei -3 °C gerührt und anschließend innerhalb einer Stunde auf Raumtemperatur erwärmt. Danach wurde das Reaktionsgemisch unter vermindertem Druck vom Lösungsmittel befreit. Der Rückstand wurde in Essigsäureethylester (50 ml) gelöst und mit vollentsalztem Wasser (50 ml) gewaschen. Nach Trocknen der organischen Phase über MgSO₄ wurde das Lösungsmittel unter vermindertem Druck entfernt. 3-Difluormethyl-1-methyl-1 H-pyrazol-4-ylcarbonsäureethylester wurde als Rückstand erhalten (Menge: 1,0 g; Reinheit It. GC: 80,6 % (bezogen auf 3-Isomer); Ausbeute: 66,3 %). Das Isomerenverhältnis von 3-Isomer zu 5-Isomer im Rückstand betrug 98 : 2.

### Herstellungsbeispiel III.6: 3-Difluormethyl-1 H-pyrazol-4-yl-carbonsäuremethylester

Zu einer Lösung von Hydrazinhydrat (100%ig, 4,36 g, 87 mmol) in Toluol (150 ml) wurde bei -50°C eine Lösung von 4,4-Difluor-3-oxo-2-(1-piperidin-1-ylmethyliden)buttersäuremethylester (97,9%ig, 20,0 g, 79 mmol) in Toluol (150 ml) tropfenweise zugegeben. Das Reaktionsgemisch wurde 2 h bei -50°C gerührt und anschließend innerhalb einer Stunde auf Raumtemperatur erwärmt. Das Reaktionsgemisch wurde mit vollentsalztem Wasser (100 ml) gewaschen. Nach Trennung der Phasen wurde die wässrige Phase einmal mit Toluol (100 ml) extrahiert. Die Toluolphasen wurden vereinigt, mit einer wässrigen ges. NaCl-Lösung (100 ml) gewaschen und anschließend am Vakuum vom Lösungsmittel befreit. Der erhaltene Rückstand (9,2 g) bestand laut GC-Analyse zu 65,3 % aus 3-Difluormethyl-1 H-pyrazol-4-yl-carbonsäuremethylester (Ausbeute: 66%). ¹H-NMR (CDCl₃): δ = 3,9 (s, 3H), 7,25 (t, 1H), 8,2 ppm (s, 1H).

### 3. Herstellung von Verbindungen der Formel (V)

### Herstellungsbeispiel V. 1: 3-Difluormethyl-1-methyl-1 H-pyrazol-4-ylcarbonsäure

Ein Gemisch aus 3-Difluormethyl-1-methyl-1 H-pyrazol-4-ylcarbonsäureethylester (1,4 g, 52 mmol) und Natronlauge (10%ig, 3,1 g, 8 mmol) wurde 2 h bei 60°C gerührt. Das Reaktionsgemisch wurde auf Raumtemperatur abgekühlt und anschließend mit konzentrierter Salzsäure auf einen pH-Wert von 1 eingestellt. Das Reaktionsgemisch wurde auf 0°C weiter abgekühlt, wobei ein Feststoff ausfiel. Der ausgefallene Feststoff wurde abfiltriert, mit Cyclohexan gewaschen und unter vermindertem Druck getrocknet. 3-Difluormethyl-1-methyl-1 H-pyrazol-4-ylcarbonsäure wurde als Feststoff (Menge: 0,8 g; Ausbeute: 87%). ¹H-NMR (DMSO-d₆): δ = 3,9 (s, 2H), 7,2 (t, 1H), 8,35 ppm (s, 1H) erhalten.

### Herstellungsbeispiel V.2: 3-Difluormethyl-1-methyl-1 H-pyrazol-4-ylcarbonsäure (konzertierte Fahrweise)

In eine Lösung von 3-Piperidin-1-ylacrylsäuremethylester (99%ig, 29,05 g, 0,17 mol) und Triethylamin (25,8 g, 0,255 mol) in Toluol (250 ml) wurde bei -30°C Difluoracetylfluorid (18,7 g, 0,187mol); 98%ig) eingeleitet. Das Reaktionsgemisch wurde 3 h bei -30 °C gerührt und anschließend innerhalb einer Stunde auf Raumtemperatur erwärmt. Das Reaktionsgemisch wurde mit vollentsalztem Wasser (250 ml) gewaschen. Nach Trennung der Phasen wurde die wässrige Phase einmal mit Toluol (200 ml) extrahiert. Die Toluolphasen wurden vereinigt und am Vakuum auf etwa 350 ml eingeengt. Die so erhaltene Reaktionslösung wurde bei -50 °C zu einer Lösung von Methylhydrazin (8,78 g, 0,187mol) in Toluol (150 ml) tropfenweise zugegeben. Das Reaktionsgemisch wurde 2 h bei -50°C gerührt und anschließend innerhalb einer Stunde auf Raumtemperatur erwärmt. Danach wurde das Reaktionsgemisch mit wässrigen Natronlauge (10%ig, 105,3g, 0,263mol) versetzt und 2 h unter Rückflussbedingungen erhitzt. Nach Abkühlen auf Raumtemperatur wurden die Phasen getrennt. Die Toluolphase wurde mit wässriger Natronlauge (10%ig, 100 ml) extrahiert. Die wässrigen Phasen wurden vereinigt, mit Salzsäure (konz.) auf einen pH-Wert von 1 eingestellt und auf 0°C abgekühlt. Der ausgefallene Feststoff wurde abfiltriert, mit Cyclohexan gewaschen und bei 60°C am Vakuum getrocknet. 3-Difluormethyl-1-methyl-1 H-pyrazol-4-yl-carbonsäure wurde in einer Menge von 21,1 g (65% Ausbeute) mit einer Reinheit von 91 % (It. quant. HPLC) erhalten.
¹H-NMR (CDCl₃): δ = 3,85 (s, 2H), 3,95 (s, 3H), 7,12 (t, 1 H), 7,9 ppm (s, 1 H).

## Patentansprüche

1. Verfahren zur Herstellung von Verbindungen der Formel (I), 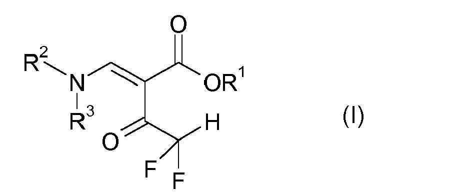 worin
R¹ für C₁-C₆-Alkyl, C₁-C₆-Haloalkyl, C₂-C₆-Alkenyl, C₃-C₁₀-Cycloalkyl oder Benzyl steht, wobei die beiden letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen;
R² gemeinsam mit R³ und dem Stickstoffatom, an das die beiden Reste gebunden sind, für einen gegebenenfalls substituierten 5- bis 10-gliedrigen heterocyclischen Rest stehen, der neben dem Stickstoffatom weitere 1, 2 oder 3 Heteroatome ausgewählt unter O, N und S als Ringglieder umfassen kann;
bei dem man
eine Verbindung der Formel (II), 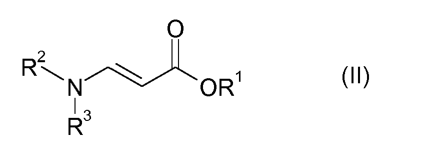 worin R¹, R² und R³ eine der zuvor gegebenen Bedeutungen aufweisen;
mit Difluoracetylfluorid umsetzt.

2. Verfahren nach Anspruch 1, worin NR²R³ für Pyrrolidin-1-yl, Oxazolidin-3-yl, 3-Methylimidazolin-1-yl, Piperidin-1-yl, Morpholin-4-yl oder 4-Methylpiperazin-1-yl steht.

3. Verfahren nach einem der vorhergehenden Ansprüche, wobei die Umsetzung der Verbindung der Formel (II) mit Difluoracetylfluorid in einem organischen Lösungsmittel mit einem Wassergehalt von weniger als 500 ppm erfolgt.

4. Verfahren nach einem der Ansprüche 1 bis 3, bei dem man die Verbindung der Formel (II) mit Difluoracetylfluorid ohne Zusatz einer Base umsetzt.

5. Verfahren nach einem der Ansprüche 1 bis 3, bei dem man die Verbindung der Formel (II) mit Difluoracetylfluorid in Gegenwart einer Base umsetzt.

6. Verfahren nach Anspruch 5, worin die Base ausgewählt ist unter tertiären Aminen.

7. Verbindungen der Formel (I), 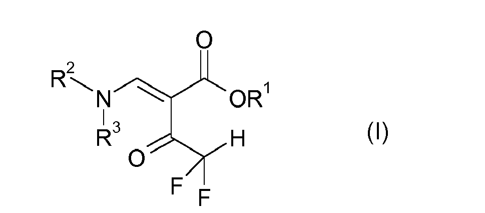 worin R¹ eine der zuvor gegebenen Bedeutungen aufweist und R² gemeinsam mit R³ und dem Stickstoffatom, an das die beiden Reste gebunden sind, für einen gegebenenfalls substituierten 5- bis 10-gliedrigen heterocyclischen Rest stehen, der neben dem Stickstoffatom weitere 1, 2 oder 3 Heteroatome ausgewählt unter O, N und S als Ringglieder umfassen kann.

8. Verfahren zur Herstellung difluormethylsubstituierter Pyrazol-4-ylcarbonsäureester der Formel (III), 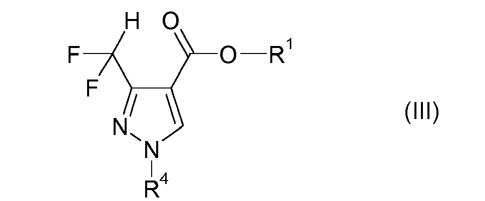 worin
R¹ für C₁-C₆-Alkyl, C₁-C₆-Haloalkyl, C₂-C₆-Alkenyl, C₃-C₁₀-Cycloalkyl oder Benzyl steht, wobei die beiden letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen; und
R⁴ für Wasserstoff, C₁-C₆-Alkyl, C₁-C₆-Haloalkyl, C₃-C₁₀-Cycloalkyl, Phenyl oder Benzyl steht, wobei die drei letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen;
bei dem man
a) eine Verbindung der Formel (I) nach einem Verfahren der Ansprüche 1 bis 6 bereitstellt, 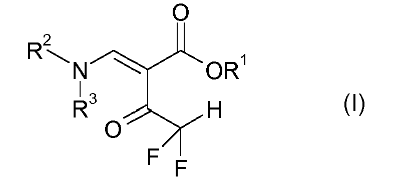 worin R¹ eine der zuvor gegebenen Bedeutungen aufweist und R² gemeinsam mit R³ und dem Stickstoffatom, an das die beiden Reste gebunden sind, für einen gegebenenfalls substituierten 5- bis 10-gliedrigen heterocyclischen Rest stehen, der neben dem Stickstoffatom weitere 1, 2 oder 3 Heteroatome ausgewählt unter O, N und S als Ringglieder umfassen kann; und
b) die in Schritt a) bereitgestellte Verbindung der Formel (I) mit einer Hydrazin-Verbindung der Formel (IV) umsetzt,
R⁴HN-NH₂ (IV)
worin R⁴ eine der zuvor gegebenen Bedeutungen aufweist.

9. Verfahren nach Anspruch 8, bei dem R⁴ in den Verbindungen der Formeln (III) und (IV) für C₁-C₄-Alkyl steht.

10. Verfahren nach Anspruch 8 oder 9, bei dem man die Verbindung der Formel (I) in Form eines durch ein Verfahren nach einem der Ansprüche 1 bis 6 erhaltenen Reaktionsgemisches ohne vorherige Isolierung der Verbindung der Formel (I) bereitstellt.

11. Verfahren nach einem der Ansprüche 8 bis 10, wobei die Umsetzung der Verbindung der Formel (I) mit der Hydrazin-Verbindung der Formel (IV) im Wesentlichen wasserfrei erfolgt.

12. Verfahren nach einem der Ansprüche 8 bis 10, wobei die Umsetzung der Verbindung der Formel (I) mit der Hydrazin-Verbindung der Formel (IV) in Gegenwart von Wasser erfolgt.

13. Verfahren zur Herstellung einer Pyrazol-4-carbonsäure der Formel (V), 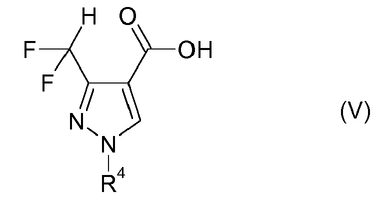 worin R⁴ eine der zuvor gegebenen Bedeutungen aufweist, umfassend
die Bereitstellung einer Verbindung der Formel (III) durch ein Verfahren gemäß einem der Ansprüche 8 bis 12 und die Umsetzung der Verbindung der Formel (III) durch Hydrolyse.

14. Verfahren nach Anspruch 13, bei dem man die Verbindung der Formel (III) in Form eines durch ein Verfahren nach einem der Ansprüche 8 bis 12 bereitgestellten Reaktionsgemisches ohne vorherige Isolierung der Verbindung der Formel (III) durch Hydrolyse umsetzt.

## Claims

1. A process for preparing compounds of the formula (I) 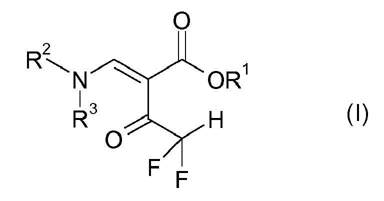 in which
R¹ is C₁-C₆-alkyl, C₁-C₆-haloalkyl, C₂-C₆-alkenyl, C₃-C₁₀-cycloalkyl or benzyl, where the two last mentioned radicals are unsubstituted or have 1, 2 or 3 substituents independently of one another selected from the group consisting of halogen, CN, nitro, C₁-C₄-alkyl, C₁-C₄-haloalkyl, C₁-C₄-alkoxy and C₁-C₄-haloalkoxy;
R² together with R³ and the nitrogen atom to which the two radicals are attached are an optionally substituted 5- to 10-membered heterocyclic radical which, in addition to the nitrogen atom, may contain a further 1, 2 or 3 heteroatoms selected from the group consisting of 0, N and S as ring members;
wherein
a compound of the formula (II) 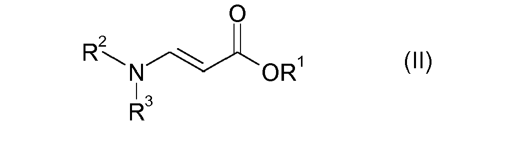 in which R¹, R² and R³ have one of the meanings given above;
is reacted with difluoroacetyl fluoride.

2. The process according to claim 1 in which NR²R³ is pyrrolidin-1-yl, oxazolidin-3-yl, 3-methylimidazolin-1-yl, piperidin-1-yl, morpholin-4-yl or 4-methylpiperazin-1-yl.

3. The process according to either of the preceding claims where the reaction of the compound of the formula (II) with difluoroacetyl fluoride is carried out in an organic solvent having a water content of less than 500 ppm.

4. The process according to any of claims 1 to 3 wherein the compound of the formula (II) is reacted with difluoroacetyl fluoride without addition of a base.

5. The process according to any of claims 1 to 3 wherein the compound of the formula (II) is reacted with difluoroacetyl fluoride in the presence of a base.

6. The process according to claim 5 where the base is selected from tertiary amines.

7. A compound of the formula (I) 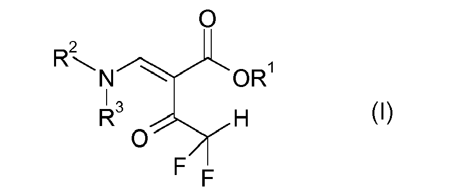 in which R¹ has one of the meanings given above and R² together with R³ and the nitrogen atom to which the two radicals are attached are an optionally substituted 5- to 10-membered heterocyclic radical which, in addition to the nitrogen atom, may contain a further 1, 2 or 3 heteroatoms selected from the group consisting of 0, N and S as ring members.

8. A process for preparing difluoromethyl-substituted pyrazol-4-ylcarboxylic esters of the formula (III) 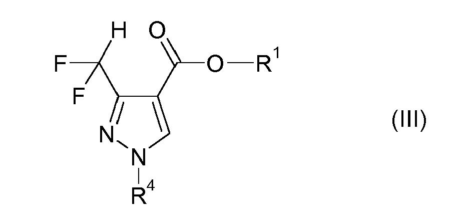 in which
R¹ is C₁-C₆-alkyl, C₁-C₆-haloalkyl, C₂-C₆-alkenyl, C₃-C₁₀-cycloalkyl or benzyl, where the two last mentioned radicals are unsubstituted or have 1, 2 or 3 substituents independently of one another selected from the group consisting of halogen, CN, nitro, C₁-C₄-alkyl, C₁-C₄-haloalkyl, C₁-C₄-alkoxy and C₁-C₄-haloalkoxy; and
R⁴ is hydrogen, C₁-C₆-alkyl, C₁-C₆-haloalkyl, C₃-C₁₀-cycloalkyl, phenyl or benzyl where the three last mentioned radicals are unsubstituted or have 1, 2 or 3 substituents independently of one another selected from the group consisting of halogen, CN, nitro, C₁-C₄-alkyl, C₁-C₄-haloalkyl, C₁-C₄-alkoxy and C₁-C₄-haloalkoxy;
wherein
a) a compound of the formula (I) is provided by a process of claims 1 to 6, 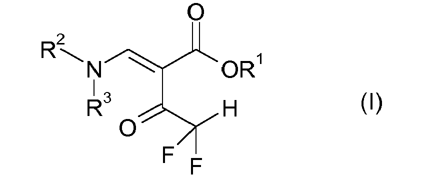 in which R¹ has one of the meanings given above and R² together with R³ and the nitrogen atom to which the two radicals are attached are an optionally substituted 5- to 10-membered heterocyclic radical which, in addition to the nitrogen atom, may contain a further 1, 2 or 3 heteroatoms selected from the group consisting of 0, N and S as ring members; and
b) the compound of the formula (I) provided in step a) is reacted with a hydrazine compound of the formula (IV)
R⁴HN-NH₂ (IV)
in which R⁴ has one of the meanings given above.

9. The process according to claim 8 wherein R⁴ in the compounds of the formulae (III) and (IV) is C₁-C₄-alkyl.

10. The process according to claim 8 or 9 wherein the compound of the formula (I) is provided in the form of a reaction mixture obtained by a process according to any of claims 1 to 6, without prior isolation of the compound of the formula (I).

11. The process according to any of claims 8 to 10 where the reaction of the compound of the formula (I) with the hydrazine compound of the formula (IV) is carried out essentially anhydrously.

12. The process according to any of claims 8 to 10 where the reaction of the compound of the formula (I) with the hydrazine compound of the formula (IV) is carried out in the presence of water.

13. A process for preparing a pyrazol-4-carboxylic acid of the formula (V) 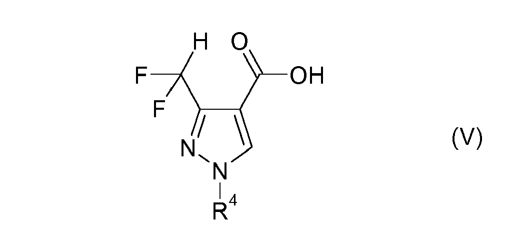 in which R⁴ has one of the meanings given above, comprising
the provision of a compound of the formula (III) by a process according to any of claims 8 to 12 and the hydrolysis of the compound of the formula (III).

14. The process according to claim 13 wherein the compound of the formula (III) is hydrolyzed in the form of a reaction mixture provided by a process according to any of claims 8 to 12, without prior isolation of the compound of the formula (III).

## Revendications

1. Procédé pour la préparation de composés de formule (I) 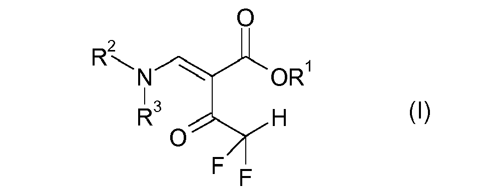 dans laquelle
R¹ représente C₁-C₆-alkyle, C₁-C₆-halogénoalkyle, C₂-C₆-alcényle, C₃-C₁₀-cycloalkyle ou benzyle, les deux derniers radicaux mentionnés étant non substitués ou présentant 1, 2 ou 3 autres substituants, choisis indépendamment les uns des autres parmi halogène, CN, nitro, C₁-C₄-alkyle, C₁-C₄-halogénoalkyle, C₁-C₄-alcoxy et C₁-C₄-halogénoalcoxy ;
R² représente, ensemble avec R³ et l'atome d'azote auquel les deux radicaux sont liés, un radical hétérocyclique le cas échéant substitué, de 5 à 10 chaînons, qui peut comprendre, outre l'atome d'azote, 1, 2 ou 3 hétéroatomes choisis parmi 0, N et S comme chaînons de cycle ;
dans lequel on transforme un composé de formule (II) 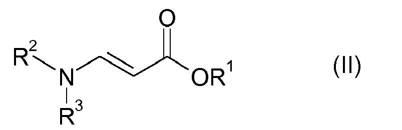 dans laquelle R¹, R² et R³ présentent une des significations indiquées ci-dessus ; avec du fluorure de difluoroacétyle.

2. Procédé selon la revendication 1, dans lequel NR²R³ représente pyrrolidin-1-yle, oxazolidin-3-yle, 3-méthylimidazolin-1-yle, pipéridin-1-yle, morpholin-4-yle ou 4-méthylpipérazin-1-yle.

3. Procédé selon l'une quelconque des revendications précédentes, la transformation du composé de formule (II) avec du fluorure de difluoroacétyle ayant lieu dans un solvant organique présentant une teneur en eau inférieure à 500 ppm.

4. Procédé selon l'une quelconque des revendications 1 à 3, dans lequel on transforme le composé de formule (II) avec du fluorure de difluoroacétyle sans addition de base.

5. Procédé selon l'une quelconque des revendications 1 à 3, dans lequel on transforme le composé de formule (II) avec du fluorure de difluoroacétyle en présence d'une base.

6. Procédé selon la revendication 5, dans lequel la base est choisie parmi les amines tertiaires.

7. Composés de formule (I) 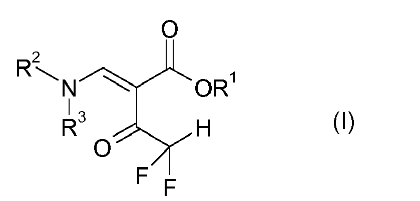 dans laquelle R¹ présente une des significations indiquées ci-dessus et R² représente, ensemble avec R³ et l'atome d'azote auquel les deux radicaux sont liés, un radical hétérocyclique le cas échéant substitué, de 5 à 10 chaînons, qui peut comprendre, outre l'atome d'azote, 1, 2 ou 3 hétéroatomes choisis parmi 0, N et S comme chaînons de cycle.

8. Procédé pour la préparation d'esters de l'acide pyrazol-4-ylcarboxylique substitués par difluorométhyle de formule (III) 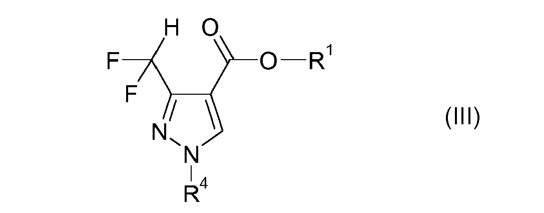 dans laquelle
R¹ représente C₁-C₆-alkyle, C₁-C₆-halogénoalkyle, C₂-C₆-alcényle, C₃-C₁₀-cycloalkyle ou benzyle, les deux derniers radicaux mentionnés étant non substitués ou présentant 1, 2 ou 3 substituants, choisis indépendamment les uns des autres parmi halogène, CN, nitro, C₁-C₄-alkyle, C₁-C₄-halogénoalkyle, C₁-C₄-alcoxy et C₁-C₄-halogénoalcoxy ; et
R⁴ représente hydrogène, C₁-C₆-alkyle, C₁-C₆-halogénoalkyle, C₃-C₁₀-cycloalkyle, phényle ou benzyle, les trois derniers radicaux mentionnés
étant non substitués ou présentant 1, 2 ou 3 substituants, choisis indépendamment les uns des autres parmi halogène, CN, nitro, C₁-C₄-alkyle, C₁-C₄-halogénoalkyle, C₁-C₄-alcoxy et C₁-C₄-halogénoalcoxy ;
dans lequel
a) on prépare un composé de formule (I) selon un procédé des revendications 1 à 6 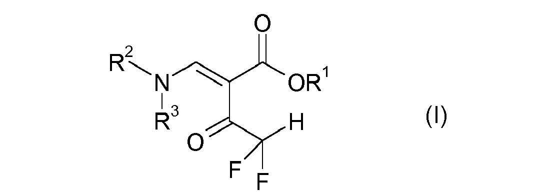 dans laquelle R¹ présente une des significations indiquées ci-dessus et R² représente, ensemble avec R³ et l'atome d'azote auquel les deux radicaux sont liés, un radical hétérocyclique le cas échéant substitué, de 5 à 10 chaînons, qui peut comprendre, outre l'atome d'azote, 1, 2 ou 3 autres hétéroatomes choisis parmi 0, N et S comme chaînons de cycle ; et
b) on transforme le composé de formule (I) préparé dans l'étape a) avec un composé d'hydrazine de formule (IV)
R⁴HN-NH₂ (IV)
dans laquelle R⁴ présente une des significations indiquées ci-dessus.

9. Procédé selon la revendication 8, dans lequel R⁴ dans les composés des formules (III) et (IV) représente C₁-C₄-alkyle.

10. Procédé selon la revendication 8 ou 9, dans lequel on prépare le composé de formule (I) sous forme d'un mélange réactionnel obtenu par un procédé selon l'une quelconque des revendications 1 à 6 sans isolement préalable du composé de formule (I).

11. Procédé selon l'une quelconque des revendications 8 à 10, la transformation du composé de formule (I) avec le composé d'hydrazine de formule (IV) ayant lieu de manière essentiellement anhydre.

12. Procédé selon l'une quelconque des revendications 8 à 10, la transformation du composé de formule (I) avec le composé d'hydrazine de formule (IV) ayant lieu en présence d'eau.

13. Procédé pour la préparation d'un acide pyrazole-4-carboxylique de formule (V) 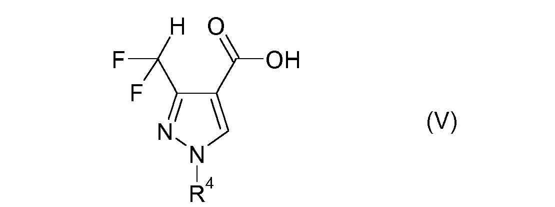 dans laquelle R⁴ présente une des significations indiquées ci-dessus, comprenant
la préparation d'un composé de formule (III) par un procédé selon l'une quelconque des revendications 8 à 12 et la transformation du composé de formule (III) par hydrolyse.

14. Procédé selon la revendication 13, dans lequel on transforme par hydrolyse le composé de formule (III) sous forme d'un mélange réactionnel préparé par un procédé selon l'une quelconque des revendications 8 à 12 sans isolement préalable du composé de formule (III).
